(19) Europäisches Patentamt
European Patent Office
Office européen des brevets

(11) **EP 4 463 193 B1**

(12) # EUROPEAN PATENT SPECIFICATION

(45) Date of publication and mention of the grant of the patent:
**05.08.2026 Bulletin 2026/32**

(21) Application number: **23700723.2**

(22) Date of filing: **11.01.2023**

(51) International Patent Classification (IPC):
***A61L 27/48*** *(2006.01)* ***A61L 27/52*** *(2006.01)*
***A61L 27/54*** *(2006.01)*

(52) Cooperative Patent Classification (CPC):
(C-Sets available)
**A61L 27/52; A61L 27/48; A61L 27/54;**
A61L 2300/64 (Cont.)

(86) International application number:
**PCT/EP2023/050516**

(87) International publication number:
**WO 2023/135155 (20.07.2023 Gazette 2023/29)**

(54) **PHOTOCURABLE PHASE-SEPARATING HYDROGELS**

PHOTOHÄRTBARE PHASENTRENNENDE HYDROGELE

HYDROGELS À SÉPARATION DE PHASES PHOTODURCISSABLES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **12.01.2022 EP 22151244**

(43) Date of publication of application:
**20.11.2024 Bulletin 2024/47**

(73) Proprietor: **ETH Zurich**
**8092 Zurich (CH)**

(72) Inventors:
- **MÜLLER, Ralph**
  **8704 Herrliberg (CH)**
- **MUELLER, Monica**
  **8048 Zürich (CH)**
- **QIN, Xiao-Hua**
  **8152 Opfikon (CH)**
- **QIU, Wanwan**
  **8046 Zürich (CH)**

(74) Representative: **Schulz Junghans Patentanwälte PartGmbB**
**Großbeerenstraße 71**
**10963 Berlin (DE)**

(56) References cited:
- **FREDERIK MAYER ET AL: "3D Two-Photon Microprinting of Nanoporous Architectures", ADVANCED MATERIALS, VCH PUBLISHERS, DE, vol. 32, no. 32, 30 June 2020 (2020-06-30), pages n/a, XP071875798, ISSN: 0935-9648, DOI: 10.1002/ADMA.202002044**
- **BROGUIERE: "Macroporous hydrogels derived from aqueous dynamic phase separation", 1 January 2019 (2019-01-01), XP093033202, Retrieved from the Internet <URL:https://doi.org/10.1016/j.biomaterials.2019.01.04> [retrieved on 20230320], DOI: 10.1002/adma.202002044**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61L 27/48, C08L 5/02;**
**A61L 27/48, C08L 5/04;**
**A61L 27/48, C08L 5/08;**
**A61L 27/48, C08L 29/04**

## Description

### Field

The invention relates to compositions comprising two polymer species, one susceptible to photocrosslinking, the other one not susceptible to photo-crosslinking. The compositions according to the invention can be used to generate macroporous hydrogels allowing the engraftment of cells. The invention further provides a method to manufacture hydrogels and implants derived from the compositions of the invention, as well as the implants themselves.

### Background

In the last decade, macroporous scaffolds have emerged as promising materials for 3D cell culture and tissue engineering since they facilitate solute transport and cell-cell communication.

A major challenge in additive manufacturing of structures useful for biological applications is the shortage of bio-resin formulations that allow digital fabrication of anatomically shaped hydrogel constructs with both macroscopically and microscopically controlled architecture to support cell infiltration, 3D cell growth and tissue regeneration. Existing hydrogel systems for 3D bioprinting often have very small pore sizes (5 nm - 50 nm) that fail to provide a permissive environment for encapsulated cells. A combination of 3D printing and phase separation has been explored in other materials than hydrogels such as glass and acrylic monomers using digital light processing (Moore et al. Nat Mater 2020, 19 (2), 212-217; Dong et al. Nature Communications 2021, 12 (1), 247) and two-photon polymerization (Mayer et al. Advanced Materials 2020, 32 (32), 2002044). The problem with these materials is that the porogen needs to be removed by vacuum drying. Very recently, a photo-curable emulsion has been used for stereolithographic printing of porous ceramics (Kleger et al. Scientific Reports 2021, 11 (1), 22316). Yet, all these materials are unsuitable for 3D bioprinting of macroporous hydrogels in the presence of living cells.

Broguiere et al., Biomaterials 2019, Vol 200, 56-65 discloses a method for encapsulation of cells into porous structures created by phase separation between PEG and highly viscous polysaccharides.

Two-photon stereolithography is disclosed in WO2017001451A1 and WO2015002707A1.

Based on the above-mentioned state of the art, the objective of the present invention is to provide means and methods to fabricate biocompatible macroporous hydrogels that support 3D cell engraftment. This objective is attained by the subject-matter of the independent claims of the present specification, with further advantageous embodiments described in the dependent claims, examples, figures and general description of this specification.

### Summary of the Invention

In one aspect, the invention relates to a composition comprising the following components in aqueous solution:

a. a first polymer susceptible to photo-crosslinking, a photoinitiator and optionally, a crosslinking agent; wherein the first polymer is functionalized with double-bond-containing moieties selected from norbornene, acrylate, vinyl ester, vinyl ether, vinyl carbonate, allyl ether, methacrylate, vinyl sulfone, and maleimide;
and

b. a second polymer not susceptible to photo-crosslinking wherein the second polymer is a sulfated or non-sulfated polysaccharide.

The first polymer and the second polymer are miscible in aqueous solution when the first polymer is not crosslinked. The mixture comprising, or consisting of the first polymer and the second polymer undergoes phase separation, separating said first and said second polymer into separate phases, if the first polymer is crosslinked.

In another aspect, the invention relates to a method to generate a three-dimensional hydrogel object, particularly an implant, comprising the following steps:

a. providing a composition according to the invention as disclosed as the first aspect of the invention or any one of its embodiments discussed below or provided in the examples;

b. sequentially illuminating different adjacent portions of the composition with light to provide the three-dimensional hydrogel object.

The invention further relates to a hydrogel object, particularly an implant, generated from the composition according to the invention, particularly as rendered by the method described herein.

One salient application of the invention is its use in 3D biofunctionalization via tomographic photopatterning.

In summary, the composition has 2 or more components. An exemplary composition contains:

1) norbornene PVA, 2) dextran sulfate, 3) a dithiol crosslinker, and 4) a radical photoinitiator in aqueous solution. Optionally, the composition may comprise a viscosity enhancer, such as gelatin.

*Terms and definitions*

For purposes of interpreting this specification, the following definitions will apply and whenever appropriate, terms used in the singular will also include the plural and vice versa. In the event that any definition set forth below conflicts with any document referenced herein, the definition set forth shall control.

The terms "comprising," "having," "containing," and "including," and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *photopolymerization-induced phase separation (PIPS)* describes a process that starts with an initially miscible solution of two polymers which has a negative Gibbs free energy. As the second law of thermodynamics states, the Gibbs free energy $\Delta G^{mix}$ is given by the following equation:

$$\Delta G^{mix} = \Delta H^{mix} - T\Delta S^{mix}$$

where $\Delta H^{mix}$ is the enthalpy, T is the temperature, and $\Delta S^{mix}$ is the entropy. Upon polymerization of the crosslinkable polymer, the entropy decreases so that the Gibbs free energy increases until it becomes positive and the two polymers phase separate.

The term *engraftment of cells* in the context of the present specification relates to three-dimensional (3D) cell growth and infiltration at the implantation site.

The term *photoinitiator* in the context of the present specification relates to any compound capable of initiating a polymerization reaction when triggered by electromagnetic radiation, particularly by light in the visible or near infrared or near UV spectrum. Non-limiting examples of photoinitiators useful to practice the current invention include single photon initiators such as Li-phenyl-2,4,6trimethylbenzoylphosphinate, 2-hydroxy-4'-(2-hydroxyethoxy)-2-methylpropiophenone (Irgacure 2959), Eosin Yellow + triethanol amine, bisacylphosphineoxide (BAPO) salts such as BAPO-ONa and BAPO-OLi; TPO (diphenyl(2,4,6-trimethylbenzoyl)phosphine oxide) lithium or sodium, VA-086 (2,2'-azobis[2-methyl-N-(2-hydroxyethyl)propionamide]; CAS No. 61551-69-7), and two-photon initiators exemplified by P2CK (3,3'-((((1E,1'E)-(2-oxocyclopentane-1,3-diylidene)bis(methanylylidene))bis(4,1-phenylene))bis(methylazanediyl))dipropanoate), G2CK (sodium 2,2'-((((1E,1'E)-(5-methyl-2-oxocyclohexane-1,3-diylidene)bis(methanylylidene))bis(4,1-phenylene))bis(methylazanediyl))diacetate), DAS (tetrapotassium 4,4'-(1,2-ethenediyl)bis[2-(3-sulfophenyl)diazenesulfonate])

See also Benedikt et al., J. Polymer Science Part A Polymer Chemistry (2015) Vol. 54, 473-479; https://doi.ora/10.1002/pola.27903 and Tromayer et al., Polymer Chemistry (2018) 9, 3108-3117; https://doi.org/10.1039/C8PY00278A.

The term *polymer susceptible to photo-crosslinking* in the context of the present specification relates to a polymer wherein each chain comprises reactive moieties that can be a) activated by light to react to form covalent bonds with

another chain directly or through a crosslinking agent, or b) form covalent bonds with a crosslinking agent that can be activated by light to form covalent bonds to the reactive moieties.

The term *crosslinking agent* in the context of the present specification relates to a bifunctional reagent, particularly a short alkyl (particularly a *$C_1$-$C_6$ alkyl),* oxyalkyl or peptide chain. The crosslinking agent comprises at least two moieties that can a) be activated by light to generate a reactive species capable of forming a covalent bond to a reactive moiety on a polymer susceptible to photo-crosslinking, or b) form a covalent bond to a reactive moiety on a polymer susceptible to photo-crosslinking activated by light.

The term *dithiol crosslinking agent* in the context of the present specification relates to a crosslinking agent comprising at least two thiol (SH) moieties that can react with the nPVA or equivalent moieties in the first polymer. A *dithiol crosslinking agent* encompasses multifunctional thiols, including linear divalent linkers exemplified by dithiols such as HS-PEG-SH, trithiols, and also encompasses macrothiols and thiol-functionalized polyvinyl alcohol (such as a high Mw PVA macrothiol), and thiol-functionalized multi-arm polyethylene glycol (particularly a poly(ethyleneglycol) scaffold having 4 or 8 SH functionalities.

A *$C_1$-$C_6$ alkyl* in the context of the present specification relates to a saturated linear or branched hydrocarbon having 1, 2, 3, 4, 5 or 6 carbon atoms, wherein one carbon-carbon bond may be unsaturated and/or one $CH_2$ moiety may be exchanged for oxygen (ether bridge) or nitrogen (NH, or NR with R being methyl, ethyl, or propyl; amino bridge). (w/w) means weight per weight and is used synonymously with (m/m), which means mass per mass. (w/v) means weight per volume.

## Detailed Description of the Invention

A first aspect of the invention relates to a composition. This composition comprises, or essentially consists of, the following components in aqueous solution:

a. a first polymer susceptible to photo-crosslinking, a photoinitiator and optionally, a crosslinking agent, wherein the first polymer is functionalized with double-bond-containing moieties selected from norbornene, acrylate, vinyl ester, vinyl ether, vinyl carbonate, allyl ether, methacrylate, vinyl sulfone, and maleimide;
and

b. a second polymer not susceptible to photo-crosslinking, wherein the second polymer is a sulfated or non-sulfated polysaccharide.

The first polymer and the second polymer are miscible and form a single phase when the first polymer is not crosslinked. Upon crosslinking of the first polymer, the mixture comprised of the first polymer and the second polymer undergoes phase separation, separating said first and said second polymer into separate phases.

Besides a photoinitiator, which is essential for three-dimensional printing (3DP), the crosslinking can also be initiated by redox or enzymatic initiating systems such as ammonium persulfate (APS)/1,2-bis(dimethylamino)ethane (TEMED) and horseradish peroxidase (HRP), or by the crosslinker/polymer (without initiator), as long as a step-growth crosslinking can proceed. The 3D printing methods and compositions of the current invention however do require a photoinitiator.

The inventors propose that expanding on the examples shown herein, it is also possible to work without crosslinking agent, e.g. using moieties that can undergo direct linkage, e.g. by chain-growth polymerization. Such moieties include, without being limited to, the group comprised of acrylates, methacrylates, vinyl esters, vinyl carbonates, vinyl carbamates, vinyl sulfones.

Other moieties useful in this context include thiols for disulfide crosslinking (see Xiao et al., Biomacromolecules 2002, 3, 6, 1304-1311). Coumarin-functionalized polymers for photodimerization/cycloaddition may similarly be employed (Urciuolo et al. Nat Biomed Eng 4, 901-915 (2020)). Dityrosine and tyramine crosslinking may also be useful (Partlow et al., ACS Biomater. Sci. Eng. 2016, 2, 12, 2108-2121; Loebel et al. Biomacromolecules 2017, 18, 3, 855-864).

### *The first polymer*

In addition to norbornene-comprising polymers, such as norbornene-functionalized PVA, double-bond ("ene") functions that are easily susceptible to thiol-ene photopolymerization include, but are not limited to the group comprising acrylate, methacrylate, vinyl ester, vinyl carbonate, allyl ether, and norbornene-functionalized multiarm poly(ethylene glycol) (PEG).

One crosslinking agent of particular utility is PEG dithiol, which was used predominantly as a crosslinker in developing the invention, where 2kDa - 5 kDa linker sizes worked well, in particular, 2 kDa and 3.4 kDa. The skilled person readily understands that multifunctional thiols (expemplified by, but not limited to, tri- or tetrathiols) might similarly be

employed.

In certain embodiments, the first polymer is functionalized with double bond containing moieties selected from the group comprising norbornene, acrylate, vinyl ester, vinyl ether, vinyl carbonate, allyl ether, methacrylate, vinyl sulfone, maleimide.

In certain particular embodiments, the first polymer is a polyvinyl alcohol functionalized with double bond containing moieties selected from the group comprising norbornene, acrylate, vinyl ester, vinyl ether, vinyl carbonate, allyl ether.

The PIPS principle is not restricted to double bond containing moieties, but can be used with any other "clickable" moiety amenable to rapid, photo-induced reaction with a partner provided in the composition leading to phase separation. Useful pairs of moieties undergoing "click chemistry" or "clickable" reactions in response to illumination include: alkyne/azide, tetrazole/alkyne or tetrazole/alkene, thiol/yne and thiol-Michael (thiol addition to a,ß unsaturated carbonyl). See Scinto et al. Bioorthogonal chemistry. Nat Rev Methods Primers 1, 30 (2021). https://doi.orq/10.1038/s43586-021-00028-z; Adzima et al. Nature Chem 3, 256-259 (2011). https://doi.orq/10.1038/nchem.980; Peng et al., Chem. Mater. 2014, 26, 23, 6819-6826; Zhang et al. ACS Macro Lett. 2016, 5, 2, 229-233).

In certain embodiments, the first polymer is functionalized with non-alkene moieties selected from the group comprising azide, alkyne, tetrazole, sulfhydryl, coumarin, tyrosine, tyramine.

In certain embodiments, the first polymer has a molecular mass of 3 kDa - 1000 kDa, particularly of 5 kDa - 200 kDa, more particularly in the range of 10 kDa - 80 kDa.

In certain embodiments, the first polymer has a molecular mass of 3 kDa - 80 kDa, particularly of 5 kDa - 60 kDa, more particularly in the range of 20 kDa - 60 kDa.

In certain particular embodiments, the first polymer is a norbornene-functionalized polyvinyl alcohol, and a dithiol crosslinking agent is present in the composition.

Shorter crosslinkers such as dithiothreitol (DTT) or other macrothiols with 3 or more -SH groups (PEG tetra-thiol, or PVA macrothiols) are also expected to work well in practicing the invention.

In certain embodiments, the norbornene-functionalized polyvinyl alcohol is a polyvinylalcohol-cis-5-norbornene-2,3-dicarboxylic acid ester.

Both the endo- and exo-conformers of the norbomene-2,3-dicarboxylic acid may be employed.

In certain embodiments, after modification, one carboxyl group is esterified with the -OH groups of PVA, while the other carboxyl group is not esterified, for neutralization to become a sodium salt (or other salts). This is important to keep good water solubility of the modified PVA.

Exemplary modifications are shown in the following scheme:

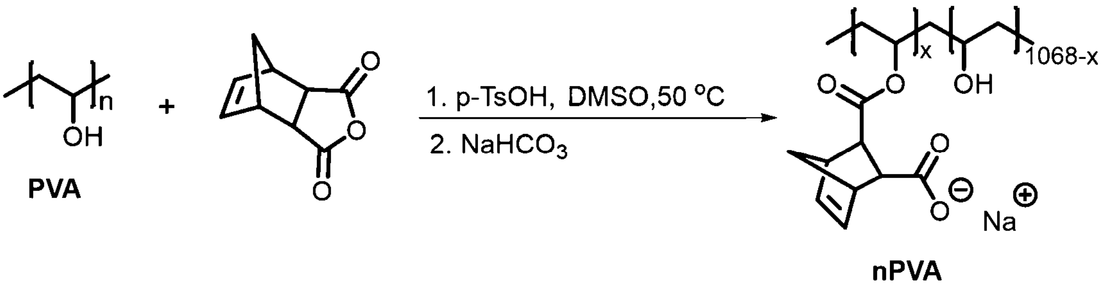


The synthesis of norbornene PVA has been reported in Qin et al. (2015) Advanced Functional Materials 25, 6606-6617 https://doi.org/10.1002/adfm.201501637; Baudis et al. (2016), J Polymer Science Part A, 54 2060-2070 https://doi.org/10.1002/pola.28073; EP3344299B1, also published as US2018243465A1.

In summary, the first polymer includes, without being limited to, PVA functionalized with ene groups, where the ene refers to norbornene, acrylate, vinyl ester, vinyl ether, vinyl carbonate, allyl ether, and the like.

In certain embodiments, the first polymer susceptible to photo-crosslinking is characterized by a molecular mass in the range of 3 kDa - 1000 kDa, particularly in the range of 5 kDa - 200 kDa, more particularly in the range of 10 kDa - 80 kDa.

In certain embodiments, the norbornene-functionalized polyvinyl alcohol has one norbornene moiety per 5 to ten free hydroxy moieties.

*The second polymer*

The second polymer is a sulfated or non-sulfated polysaccharide, particularly a sulfated polysaccharide.

In certain particular embodiments, the polysaccharide is selected from the group comprised of dextran sulfate, chondroitin sulfate, sulfated alginate, dextran, mannuronan, hyaluronan, alginate, particularly wherein the polysaccharide is selected from the group comprised of dextran sulfate and chondroitin sulfate, more particularly wherein the polysaccharide is dextran sulfate.

In certain embodiments, the second polymer can be chosen to be gelatin. Gelatin is shown to contribute to the performance of some of the compositions shown in the examples, and is assigned the role of a viscosity enhancer elsewhere herein. The inventors however have been able to obtain compositions undergoing phase separation upon crosslinking, with only the first polymer and gelatin present.

In certain embodiments, the second polymer has a molecular mass of 3-500 kDa. In certain particular embodiments, the second polymer has a molecular mass of 5 kDa to 90kDa. In certain more particular embodiments, the second polymer has a molecular mass from 10 kDa to 80 kDa. In certain even more particular embodiments, the second polymer has a molecular mass from 20 to 50 kDa.

In certain embodiments, the second polymer is or comprises a dextran sulfate having a molecular mass of 3 to 500 kDa.

In certain embodiments, the second polymer is or comprises a poly(ethylene glycol). In particular embodiments thereof, the poly(ethylene glycol) has a molecular mass of 5 to 50 kDa.

Sulfation (introduction of a negative charge) promotes PIPS, as the inventors found no phase separation with the same concentration and unsulfated dextran alone. Without wanting to be bound by theory, the inventors assume that this finding can be explained by the fact that nPVA also has a negative charge.

A possible synergistic effect of molecular mass and sulfation was not investigated by the inventors. The dextran sulfate employed in the examples had a molecular mass of 40 kDa.

The inventors have found that for a given 2-polymer mixture (MW known), an approximately similar charge between the two polymers (i.e., both negative charge in nPVA and dextran sulfate) promotes phase separation.

*The crosslinker*

In certain embodiments, a *dithiol crosslinking agent* is present. A multifunctional thiol that comprises at least two SH functions that can react with double bonds, such as contained in nPVA, can be employed.

In certain particular embodiments, the crosslinker is a linear divalent linker. In certain exemplary embodiments, this is a PEG dithiol HS-PEG-SH as employed in the Examples.

In certain other embodiments, the crosslinker is a trithiol.

In certain other embodiments, the crosslinker is a macrothiol, such as a high Mw PVA macrothiol. In certain other embodiments, the crosslinker is a thiol-functionalized polyvinyl alcohol.

Multi-arm thiol crosslinkers are commercially available from different providers, one being LaysanBio (Arab, Alabama, USA).

*Concentration of polymers*

As regards concentrations of first and second polymer, the feasible range depends on the polymers used and can be determined by a phase diagram. Compositions close to the critical point and just below the binodal curve (see Fig. 8) are most suitable. More generally, the phase diagram, critical point, and the concentrations of each polymer for a miscible solution depends on the MW of each polymer.

Benmouna et al. (Macromolecules 2000, 33, 3, 960-967) have studied the influence of molecular mass on phase diagrams. Without wishing to be bound by theory, the inventors expect that the critical point (CP) moves to lower concentrations as the Mw increases. Also, there an expanded two-phase zone with increasing polymer molecular weight is expected, such as in the PEG-dextran system.

The inventors deem the applicable concentration for each polymer to cover a wide range: 0.5-40%, particularly 0.8-10%, more particularly 1.0 -7.7%. even more particularly 1.5 to 5.0% of each first and second polymer.

The polymers investigated in the examples of the present specification showed particularly advantageous results when 2-3% nPVA and 2.5-3% dextran sulfate were employed.

In certain embodiments, the second polymer is dextran sulfate at a concentration of 2.0% to 3.5% (m/m). In certain particular embodiments, the second polymer is dextran sulfate at a concentration of 2.3% to 3.2% (m/m). In certain more particular embodiments, the second polymer is dextran sulfate at a concentration of 2.5% to 3.0% (m/m).

In certain embodiments, the inventors found a stable gel could be formed with 0.8% of a higher MW norbornene PVA (nPVA; 200 kDa). In certain embodiments, the inventors found a stable gel could be formed with 1.5% nPVA of 47kDa.

In certain embodiments, the concentration of the first polymer is 0.5-50% (m/m). In certain particular embodiments, the concentration of the first polymer is 0.5-10% (m/m). In certain more particular embodiments, the concentration of the first polymer is 2-3% (m/m). In certain particular embodiments thereof, a crosslinking agent is present and the

concentration of the crosslinking agent is 1-3% (m/m).

In certain embodiments, the first polymer is nPVA and the 1st polymer's concentration is 0.5-0.8% (m/m). The optimal concentration may vary with the molecular mass of the polymer.

In certain embodiments, the composition comprises a first polymer comprising reactive ene (double bond) moieties, and the composition further comprises a crosslinking agent comprising thiol moieties, and the stoichiometric ratio of thiol moieties in the crosslinking agent to ene moieties in the first polymer is selected to range from 1:10 to 2:1. In certain particular embodiments, the stoichiometric ratio of thiol moieties in the crosslinking agent to ene moieties in the first polymer ranges from 1:5 to 2:1. In even more particular embodiments, this ratio ranges from 2:5 to 6:5.

In practical terms, a parameter of higher importance than the weight concentration, is the molar amount of the crosslinker, which should be stoichiometric to the groups in the first polymer, e.g. the thiol:ene ratio is in the range of 1:20 to 5:1. In particular embodiments, the thiol:ene ratio is selected to range from 1:10 to 2:1. In more particular embodiments, the thiol:ene ratio is selected to range from 1:5 to 2:1. In even more particular embodiments, the thiol:ene ratio is selected to range from 2:5 to 6:5.

In certain embodiments, the concentration of the second polymer is 0.1% to 10% (m/m).

In certain particular embodiments, the concentration of the second polymer is 0.5% to 10.0% (m/m). In certain more particular embodiments, the concentration of the second polymer is 0.2% to 4.5% (m/m). In certain even more particular embodiments, the concentration of the second polymer is 1% to 4.5% (m/m).

The inventors have been able to ascertain that, if a viscosity enhancer, such as for example gelatin, is present in the composition, the required concentrations for the second polymer is significantly lower. This is particularly true if the second polymer is dextran sulfate, in which case the concentrations can be selected as low as 0.1-0.2% for phase separation.

Workable concentrations of the second polymer depend on the concentration of the first polymer. The skilled person will be able to select useful ranges from the phase diagram. An exemplary phase diagram is shown in Fig. 8.

Different ways of creating a phase diagram are known in the art. One simple method is cloud point titration (see Capela et al., Chem Eng Educ. 2019 Spring; 53(2): 112-120). The workable compositions should be in the monophasic region and beneath the binodal curve shown in Fig. X (dashed line). In particular embodiments, an optimal composition is selected corresponding to the composition just below the critical point. The corresponding polymer concentration is within $\pm$ 0.5 wt% deviation from the critical point (CP).

Further adjustment might be needed if a viscosity enhancer is added. For instance, when creating hydrogels containing the viscosity enhancer with varying concentrations of the second polymer, the person skilled in the art will need to determine which gel composition stays intact after crosslinking, and if it is pore-forming. This may be explored by creating a phase diagram.

In certain embodiments, the composition further comprises a viscosity enhancer. In particular embodiments, the viscosity enhancer is selected from the group comprising gelatin, hyaluronic acid, gellan gum, poloxamers (block copolymers of ethylene oxide and propylene oxide, marketed as Pluronic®.

The amount of viscosity enhancer, if any is present, is determined by the required viscosity of the resin for a specific printing technique. For tomographic volumetric printing, the resin viscosity is critical parameter to produce high-resolution objects. One recommended viscosity value is 4 Pa s according to the manufacturer of the particular set up used in experimentation. The inventors, however, also succeeded printing with much lower viscosity than this value.

A particularly workable viscosity for tomographic printing is 0.1 - 50 Pa s at a temperature of 25 °C.

A particularly workable viscosity for direct gel casting and light-assisted extrusion printing is 0.03 - 50 kPa s at a temperature of 25 °C.

If a high Mw polymer is employed as viscosity enhancer, such as hyaluronan or PVA, much lower amounts down to 0.2%-0.5% would suffice. Notably, keeping the amount of enhancer as low as possible will minimize its effect on phase separation.

Another aspect of the invention relates to a method to generate a three-dimensional hydrogel object, particularly an implant, comprising the steps of providing a composition as set forth in the preceding aspect of the invention, or according to any one of the preceding embodiments or examples provided herein, and sequentially illuminating different adjacent portions of the composition with light to provide the three-dimensional hydrogel object.

The wavelength of the light for sequentially illuminating different adjacent portions of the composition is selected depending on the photoinitiator and can range from 250 - 1300 nm. In particular embodiments, the wavelength is selected in a range from 365 - 500 nm for single-photon excitation. In other particular embodiments, the wavelength is selected in a range from 700 - 1100 nm for multiphoton excitation.

The energy of the light can be selected from 5 mJ to 1000 mJ for UV lamps or LED lamps (single-photon excitation), or from 5 mJ to 1000 mJ for focused femtosecond laser (multiphoton excitation).

In certain embodiments, the light intensity employed ranges from 5 to 150 $mWcm^{-2}$. In particular embodiments, the intensity ranges from 95 to 125 $mWcm^{-2}$.

Without viscosity enhancer, the temperature for practicing the method of the invention is in the range of 5 °C to

50°C, particularly in 22 °C to 40 °C. If gelatin is present as viscosity enhancer, the temperature must be above 22 °C during the crosslinking, so that the phase-separating resin is in its liquid state.

As discussed above, the viscosity and the temperature will impact the printability.

If the temperature is too low, e.g. 4 °C, the composition may phase separate before printing. A temperature ranging from 10 °C to 40 °C has been found useful.

If the viscosity of the composition prior to crosslinking is too low, the printed structures will sediment during tomographic printing where the vial containing the resin is rotating. However, if the viscosity is too high, it may impede the propagation of the crosslinking. Favourable viscosity ranges are given above.

More disclosure on viscosity ranges favourable for the printing methods contemplated herein are disclosed in US20200384682A1. One such value is a dynamic viscosity between 1000 and 50000 cP (centipoises) at a temperature of 25 °C, with 1 cP = 1 mPa s.

For 3D cell culture and bioprinting applications, the temperature for practicing the method of the invention is in the range of 35 °C to 40 °C, particularly at 37 °C.

The ionic strength of the solution in which the phase separation takes place influences the phase diagram. The inventors have found that advantageous ionic strengths range from 100 mOsm to 1000 mOsm, particularly from 200 mOSm to 600 mOsm.

In certain embodiments, the pH of the composition may range from 6.0 - 8.5, particularly from 7.0 - 8.0.

Yet another aspect of the invention relates to a three-dimensional hydrogel object, particularly an implant, obtained by the method according to the invention.

In certain embodiments, the hydrogel is characterized by:

- the presence of pores. In typical embodiments, the pores are of a size of 0.2-1000 μm, particularly 500 nm - 100 μm;
- interconnectivity of 20-100%, particularly of 40-100%;
- porosity of 10-95%, particularly 40-90%;
- a stiffness of 1-1000000 Pa, particularly 10-10000 Pa, more particularly 10-5000 Pa, even more particularly 20-1000 Pa;
- viscoelasticity, wherein the half-time for stress-relaxation ($t_{1/2}$) is in the range of 5-10000 s, more particularly 10-2000 s, even more particularly 20-500 s.

Pore size can be determined by confocal laser scanning microscopy through either labelling the first polymer with a fluorescent dye or diffusion of the porous space with a fluorescently labelled polymer with known size, as described in Huebsch et al. (Nature Mater 2015, 14, 1269-1277; https://doi.org/10.1038/nmat4407) and EP3868416A1, respectively. Any pore size limitation given here shall be determined on the basis of the protocol given in that publication. Additional protocols can be found in Broguiere et al., Biomaterials 2019, Vol 200, 56-65, which may be employed as guidance where Huebsch et al. does not suffice. Based on the confocal images, the pore size can be analysed by 2D fast fourier transformation (2D-FFT), as described by Tran-Cong et al., Physical Review E 60, 2, R1150-1153 (1999).

Pore interconnectivity can be analyzed using a MATLAB script described in Vandaele et al., Soft Matter, 2020,16, 4210-4219. The following paragraphs quoted from that publications describe the method employed:
"Specifically, after segmentation, a binary image is generated, where each pixel (2D) or voxel (3D) is classified as belonging to a pore (value 0) or to a polymer (value 1), depending on its intensity (below or above a chosen intensity threshold). The binary volume created is cleaned from small artefacts as a post-processing step. This segmentation step leads to the detection of the pores. It is important to mention that pores with a volume smaller than 0.0065 $\mu m^3$ (diameter of approx. 0.12 μm) will be removed from the analysis."

Stiffness was measured according to the following protocol:
In order to determine mechanical stiffness of the hydrogels, rheological tests were performed with different gel compositions. In detail, gel precursor solutions were prepared and tested on the Anton Paar rheometer MCR302 using a PP20 plate and a glass plate as the bottom. Gels were crosslinked at 25 °C while a time-sweep, photo-rheology measurement was performed at frequency of 1 Hz, 1% strain, a gap of 100 μm, UV irradiation for 5 min. For each sample, 40 μl gel solution was loaded into the center of the glass plate and after setting the PP20 plate to the desired gap position, mineral oil was placed around the gel to prevent dehydration. The elastic modulus (G') plateau is defined as the stiffness. (Gehlen et al., bioRxiv doi.org/10.1101/2021.11.14.468504)

The inventors surprisingly observed that the gelatin-containing gels are stress-relaxing.

In certain embodiments, the hydrogel object according to the invention, further comprises live mammalian cells, particularly human mesenchymal stem cells (hMSC) and/or human umbilical vein endothelial cells (HUVEC).

For gels wherein cells are present, the cells are mixed with the precursor solution: the cells are suspended in the precursor solution (except the crosslinker). Then, the crosslinker is added and the whole solution is put under a light source (or excited by a focused laser) for bulk (or local) crosslinking. Light having a wavelength of < 340 nm is not suitable for crosslinking for any application in which cells are present, because of DNA damage.

Cells that can be advantageously employed in the invention include: induced pluripotency stem cells, adult stem cells, adipose and mesenchymal stem cells, primary and differentiated cells (osteoblasts, osteocytes, tenocytes, chondrocytes, neurons, muscle cells, fibroblasts). Other cells can similarly benefit from the microporosity.

In certain most particular embodiments, the compositions and methods of the present invention comprise human mesenchymal stem cells (hMSC) and/or human umbilical vein endothelial cells (HUVEC).

The inventors similarly envision that the compositions and methods of the present invention can be advantageously employed to cultivate other cells, particularly neurons, dermal fibroblasts, musclederived cells, and tenocytes.

In one particular, non-limiting embodiment further explored in the example section herein, a phase-separating thiol-ene photoresin is provided for light-assisted 3D-printing of hierarchical macroporous hydrogels that support 3D cell growth. The resin consists of norbornene-functionalized polyvinyl alcohol, dithiol crosslinker and dextran sulfate, which can rapidly form a hydrogel with interconnected pores by photopolymerization-induced phase separation (PIPS). The pore size is tunable in the range of 2-40 μm as quantified by fast Fourier transformation of confocal imaging data and depends on light intensity, polymer composition and molecular charge. After 3D photoencapsulation, high cell viability (>90%) is achieved and cell spreading in the macroporous hydrogels is significantly higher than in conventional nanoporous hydrogels. The incorporation of a thermo-reversible gelatin network enables its printability for tomographic volumetric bioprinting in the presence of human mesenchymal stem cells. A cm-scale cell-laden hydrogel construct with vasculature-like channels and interconnected pores can be 3D-printed in 12 seconds. The materials are cell-compatible, low-cost, easy-to-make and highly efficient for PIPS and light-based 3D-printing, which is unachievable with conventional 3D-printable hydrogels. Such materials are promising for future photofabrication of complex tissues with a hierarchical porosity within seconds.

Wherever alternatives for single separable features are laid out herein as "embodiments" or "items", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

*Description of the Figures*

Fig. 1 shows **macroporous hydrogels by photopolymerization-induced phase separation (PIPS). A)** Schematic representation of the PIPS process: two initially miscible monomers become immiscible during the photocrosslinking of one monomer because of entropy change, which induces phase separation and pore formation. **B)** Schematic of radical-mediated photoinitiated thiol-ene polymerization. **C)** Chemical structures of key components for PIPS: 1) norbornene-functionalized polyvinyl alcohol (nPVA), 2) dithiol linker, 3) dextran sulfate and 4) water-soluble photoinitiator (LAP, lithium phenyl-2,4,6-trimethylbenzoylphosphinate). C) Photographs of phase-separated hydrogels showing their optical transparency. Scale bars = 2 mm. **D)** Representative confocal microscopy image of macroporous gels permeabilized with FITC-dextran. Scale bar = 10 μm.

Fig. 2 shows **macroporous hydrogels by PIPS between norbornene-functionalized PVA (nPVA) and dextran sulfates (DS). A)** Approximation of a phase diagram at room temperature with nPVA (MW: 47 kDa) and DS (MW: 40 kDa). **B)** Time sweep of storage (G') and loss (G") modulus of Mix 1 (2% nPVA 2.5% DS) with an UV lamp (236 mW $cm^{-2}$) turned on after 60 s; 10 rad $s^{-1}$, 0.5% strain. **C)** Evidence of PIPS. Representative confocal images of Mix 1 and Mix 2 before and after UV crosslinking (18 mW $cm^{-2}$). 1/50th of FITC-labelled nPVA (red) was used for confocal imaging. **D)** Diffusion of FITC-dextran molecules into the pores. Representative confocal images of Mix 1 (2.5% DS) and Mix 2 (3% DS) crosslinked under UV irradiation (18 mW $cm^{-2}$) after 3 days of swelling in PBS and then 2 days in a FITC-dextran solution (green, indicating the void space); images from middle of gels show that the dye has penetrated the hydrogel. Scale bars: 10 μm (C, D).

Fig. 3 shows **the effect of material composition on structural and physical properties of nPVA hydrogels. A)** Molecular charge promotes the phase separation between nPVA and dextran derivatives. Representative confocal images (left) of Mix 3 containing 2% nPVA and 3.1% dextran derivatives after UV curing (18 mW $cm^{-2}$). A 1/50th of FITC-labelled nPVA (red) was used for confocal imaging. RIFFT of the images showing a clear peak corresponding to a length scale of 5 μm (arrow) with DS but not with dextran. **B)** Phase separation with 3% nPVA and different DS concentrations resulting in pores at different scales. Representative confocal images after UV curing (18 mW $cm^{-2}$). RIFFT of the images showing clear peaks (arrows) corresponding to a length scale of 35 μm (top) and 4 μm (bottom), respectively. **C-E)** In situ photorheometry of hydrogels with varying nPVA (C-D) and DS (E) concentrations; all measured with 10 rad $s^{-1}$ and 0.5% strain in humidified atmosphere. C) Representative time sweep of storage moduli (G') of gels with 2% DS and varying nPVA with UV lamp (236 mW $cm^{-2}$) turned on after 60 s. D-E) G' of gels with either 2% DS (D) or 2% nPVA (E) after 4 min of crosslinking (N=3); Brown-Forsynthe & Welch one-way ANOVA with Dunnet's multiple comparisons test, p-values: p=0.0102 (2 vs. 3% nPVA), p=0.0115 (2% vs. 5% nPVA), p=0.0258 (3% vs. 5% nPVA) , p= 0.0177 (0% vs. 2.5% DS). **F-G)** Gel turbidity is higher in phase-separated compositions close to the critical point (N=5). F) Turbidity of UV-cured (18 mW $cm^{-2}$) nPVA gels with 2.5% DS in PBS showing significantly higher turbidity with 2% nPVA compared to 5% nPVA; ordinary one-way ANOVA followed by Tukey's multiple comparisons test, ***: p= 0.0009. G) Turbidity of 4% nPVA gels with 0.5, 1 and 1.5% DS before and after UV curing (~20 mW $cm^{-2}$); two-way ANOVA followed by Tukey's multiple comparisons test, ****: p <0.0001. Scale bars: 10 μm (A, B bottom image) and 50 μm (B top image). Data presented as mean ± SD.

Fig. 4 shows **the effect of light intensity on pore size and crosslinking dynamics. A-B)** Characteristic length scale of the pores can be tuned as a function of the light intensity. A) Representative confocal images of UV-cured hydrogels (Mix 3) with light intensities ranging from 5 to 100 $mWcm^{-2}$. B) Characteristic length scale obtained from RIFFT plot peaks of such images (N=3); Brown-Forsynthe and Welch ANOVA test followed by Dunnett's multiple comparisons test with p-value **: p = 0.0093. **C-F)** Crosslinking dynamics with different light intensities: effect on the time sweep of gel storage moduli (C) and G'-slope (D), delay time for crosslinking (E) and time to reach 80% of the G'-plateau (F) under UV irradiation from 60 s at different light intensities (5-20 mW $cm^{-2}$). Data presented as mean ± SD. Scale bars = 10 μm (A).

Fig. 5 shows **3D Photoencapsulation of hMSCs in macroporous PVA hydrogels. A)** Schematic representation of UV cell encapsulation, where the gel is crosslinked under UV in the presence of live hMSC cells. **B)** Cell viability on day 1 as determined by a live-dead assay (N=3); one-way ANOVA with Tukey's correction for multiple comparisons, *: p = 0.0180, ****: p < 0.0001. **C)** Representative confocal images of live-dead (greenred) stained hMSCs on day 1, maximum intensity projections (MIP) from z-stacks (100 μm): light intensity at 20 mW $cm^{-2}$, 5 min. **D-E)** Cell morphology in nPVA hydrogels. D) Average cell area after 4 and 13 days of 3D osteogenic culture (N=3); two-way ANOVA with correction for multiple comparisons with Sidak for comparison of the two timepoints and with Tukey for comparisons of the three compositions, *: p = 0.0358, **: p = 0.0063. E) Representative confocal images of actin-nuclei-stained cells. Data presented as mean ± SD. Scale bars = 100 μm (A, E) and 20 μm (D).

Fig. 6 shows **volumetric tomographic photofabrication of macroporous hydrogels using the phase-separating resin. A)** Schematic representation of the ultrafast volumetric bioprinting technique: a cm-scale, anatomic shaped 3D object can be fabricated within 10-30 s in a rotating vial containing the photoink solution while tomographic light patterns of the 3D model are projected into the volume from multiple angles using a DLP modulator. **B)** STL model of a vascularized branch model. **C)** Photograph of the sample during the volumetric photofabrication process with the printed object marked by a rectangle. **D)** Stereomicroscope image of the printed structure after two days storage in PBS at 37°C to leach out gelatin and one day in FITC-dextran solution (green). **E)** Representative confocal image at the border of the same construct after two days in FITC-dextran. **F)** RIFFT of the image shown in shown in E) showing a clear peak (arrow) indicating a uniform length scale of 18 μm for the porosity. Scale bars: 1 mm (D) and 50 μm (E).

Fig. 7 shows **temperature-dependent gelation, complex viscosity and crosslinking of the optimized resin with 5% gelatin. A)** Temperature sweep of storage (G') and loss (G") moduli from 37 °C to 8 °C showing the temperature-dependent gelation of gelatin at around 20-22 °C. **B)** Complex viscosity obtained from the same measurements. **C)** Time sweep of G' and G" at 25 °C showing crosslinking after UV light (7.9 mW $cm^{-2}$, 365 nm) is turned on after 60 s of measuring. 2% nPVA (DoF = 20%) 1% DS 5% gelatin, drying prevented by mineral oil or wet tissue. 1 Hz, 0.5% (C) or 1% (A, B) strain, representative curves of 3 separate photorheometry experiments (N=3) shown.

Fig. 8 shows **the phase diagram and composition selection.** CP: critical point, and the region recommended for the compositions disclosed herein is indicated as the area enclosed by the red dashed lines below the

curve. Polymer 1: 1st polymer; polymer 2: 2nd polymer.

Fig. 9 shows **two-photon-induced phase separation in nPVA hydrogels. A-D)** Confocal microscopy images of the microstructures in 2% (A, C) and 3% (B, D) nPVA gels supplemented with 1% DS and 5% gelatin after 2PP and washing in PBS at 37 °C for one hour (A) and one day (B-D), respectively. Overviews of the laser parameter screening (A-B) and high magnification images (C-D) of the macroporous hydrogels with a scanning speed (SS) of 200 Hz and a laser power (LP) 40%. The green fluorescence originates from the residual two-photon photoinitiator G2CK. Scale bars: 100 μm (A-B) and 10 μm (C-D).

Fig. 10 shows **volumetric tomographic photofabrication of a perfusable cell-laden hydrogel construct. A)** A tubular CAD model for 3D bioprinting of perfusable constructs: C1, C2, C3, and P1 refer to different locations in the big channel (C1) and small channels (C2, left; C3, right), respectively. **B-C)** Confocal images of calcein AM-stained cells (green) at day 3 (B) and day 4 (C) in a construct printed from the soft bio-resin: 1.5% nPVA, PEG-2-SH (thiol:ene=4:5), 5% gelatin, 2 mM LAP and 2 Mio hMSCs per mL. Scale bars, 1 mm (left) and 200 μm (right, C1-C2). **D)** Bright-field image of cell clusters close to the big channel (C1) at day 4, scale bar, 200 μm. **E-F)** Confocal images of actin-nuclei stained cell clusters at positions C2 and C3 (E) as well as C1 (F) at day 7. Scale bars, 500 μm. **G)** High-magnification confocal images of two actin-nuclei stained cell clusters in C1 and C2, respectively. Scale bars, 100 μm. **H-I)** Confocal images of representative cells outside the channels in the printed construct. Scale bars, 100 μm (H), 20 μm (H insert), and 20 μm (I).

Fig. 11 shows **volumetric tomographic photofabrication of hetero-cellular pre-vascularized hydrogel construct. A)** Schematic of the procedure for injecting HUVECs into a hMSC-laden gel construct printed from a soft bio-resin (1.5% nPVA, PEG-2-SH, thiol:ene=4:5, 5% gelatin, 2 mM LAP, 1 Mio hMSCs per mL) with open channels at day 7 and then cultivated till the endothelial cells form a lumen. **B)** Confocal image of Dil-stained HUVECs (red) and calcein-stained hMSCs (green). Scale bar, 500 μm. **C)** Confocal image of an endothelial lumen through self-organization of seeded HUVECs at day 17. Scale bar, 100 μm.

Fig. 12 shows **volumetric tomographic photopatterning of biochemical motifs within preformed hydrogels in seconds. A)** Schematic of the procedure for 3D photografting of molecule-of-interests within preformed hydrogel networks by light-triggered thiol-ene photo-click conjugation. **B)** Example of volumetric photopatterning of a helical structure within a preformed PVA hydrogel within 16.3 s. Scale bar = 500 μm.

Fig. 13 shows **porosity in the adapted phase-separating resin after casting and volumetric bioprinting. A)** Phase-contrast microscopy images immediately after acellular gel casting of the adapted resin for volumetric bioprinting with varying amounts of DS (2% nPVA, 4% gelatin). The wavelength and intensity of light was 365 nm and 20 mW/cm$^2$, respectively. Scale bar = 100 μm. **B)** Confocal microscopy images of a volumetrically bioprinted hydrogel construct (2% nPVA, 0.2% DS, 4% gelatin) containing hMSCs, fixed following an osteogenic culture for 2 days and stained for 24 hours in FITC-dextran (green). **C)** RIFFT of the image in B) showing a peak corresponding to a length scale of 2.2 μm. Scale bar = 20 μm.

## Examples

### *Example 1: Evidence of PIPS*

To determine potential compositions for interconnected pores, an approximate phase diagram of nPVA and DS was made (Fig. 2A). It resembles a phase diagram of PVA and dextran found in literature. Assuming symmetry, the critical point was estimated to be at 2.5% DS and 2.5% nPVA. At this point, the volume contribution of both components is similar, making formation of interconnected pores. Below the binodal curve, the two polymers are miscible, while two separate phases exist in compositions above it. Most compositions were chosen just below the binodal curve, so that the material phase separates during photopolymerization.

To evaluate the crosslinking ability of the chosen composition, photorheometry was performed. Photorheometry showed rapid crosslinking within 40 seconds, as indicated by the sharp increase of the storage modulus (G') after turning on the UV source (Fig. 2B). The storage modulus after crosslinking was around 370 Pa (Mix 1 in Table 1). The photorheometry results evidenced a good reproducibility of the formulation (N=3).

To investigate if the composition phase separates upon photopolymerization, fluorescently labelled nPVA was added to the formulation (1/50th of non-labeled) as a reporter of phase separation. The samples before and after UV curing were investigated by confocal microscopy imaging. As shown in Fig. 2C, porous structures were observed only after the

UV curing. This confirms that the gel precursors were initially miscible and phase-separated during photoinduced crosslinking. Moreover, the pore morphology can be tuned by the DS concentration: i.e., 2.5% DS results in bi-continuous structures, while 3% DS results in droplets. Radial intensity plots of the Fast Fourier Transformation (RIFFT) of the images after curing show a clear peak, implying a uniform length scale. Both peaks correspond to a length scale of around 8 μm. With higher DS concentrations, the pores were inhomogeneous, which can be explained by phase separation before polymerization, as these compositions lie in the immiscible region of the phase diagram. No phase separation was found in the samples with lower DS concentrations and without DS.

Since the pore size may change dramatically after swelling, a dye diffusion experiment was performed to verify the gel microstructure. Confocal microscope imaging data showed that FITC-dextran (*Mw,* 500 kDa) could permeate the pore space (Fig. 2D). The RIFFT showed a peak for both compositions. No pores were seen in the sample without DS. The sample with 3% DS had a length scale of around 8 μm. Surprisingly, the length scale for 2.5% DS was found to be around 3 μm, which is smaller than the length scale observed before swelling with the labelled nPVA. This may be explained by a change in permeability because of swelling, which could have made the pores smaller because the gel was confined within a mold. The porosity was around 40% and no significant difference was found between 2.5% and 3% DS. To summarize, the PVA hydrogels by PIPS are permeable by FITC-dextran and the pore size can be tuned by varying the DS concentration.

*Example 2: Pore size, crosslinking and turbidity can be tuned by the composition*

To study the effect of molecular charge on PIPS, the phase-separating resin was compared to a composition with the uncharged dextran instead of DS (Table 1). The components were mixed with different stock concentrations (Mix 3). With the same concentration, no pore formation was observed in the sample with dextran while obvious pores were seen with DS (Fig. 3A). RIFFT analysis revealed that there is no peak in the sample with dextran, whereas a peak corresponding to a pore size of 5 μm was found in the sample with DS. These results imply that charged molecules promote pore formation and highlight the uniqueness of negatively charged DS for PIPS, which promotes pore formation through repelling the negatively charged nPVA.

Resins with a higher nPVA content were explored as alternative compositions. A resin containing 3% nPVA and 3% DS exhibited larger pores after PIPS (Fig. 3B) with the peak of the RIFFT corresponding to a length scale of 35 μm. With 2.5% DS, pores on a length scale of 4 μm were observed. At 3.5% DS, the composition probably phase-separated before photopolymerization. Compositions with higher nPVA concentrations were also explored, but the pores were either not interconnected or less homogenously distributed, probably because of phase separation before photopolymerization.

Photo-rheology was used to determine if physical properties of the hydrogels can be tuned by the composition. The storage modulus (G') plateau value was higher with the increase of nPVA concentration for the same thiol-ene ratio among groups (Fig. 3C-D). The mean values were 146, 1'344 and 3'186 Pa for 2, 3 and 5% nPVA, respectively. This indicates the increase of crosslinking density as the increase of polymer content. Notably, the photo-curing of a resin with 1% nPVA and 2.5% DS was unsuccessful due to insufficient crosslinking and too low polymer content.

Next, we tested if the variation of DS concentration leads to changes in gel stiffness (defined as the G'-plateau). Interestingly, the stiffness was significantly higher for the sample with 2.5% DS compared to the control without DS (Fig. 3E). This is likely due to the effect of partitioning where nPVA and PEG-2-SH as the crosslinkable components may be rich in one phase whereas DS is rich in the other phase. This may lead to a higher crosslinking density in the nPVA-rich phase, which results in a higher bulk stiffness. This is supported by no significant difference between 0% and 2% DS gels, which both did not show signs of phase separation based on confocal imaging data (data not shown). The amplitude and frequency sweeps showed that a strain of 0.5% was within the linear viscoelastic region and the gels have a solid behavior over a wide range of frequencies.

The influence of gel composition on turbidity of the hydrogels was studied on a plate reader. The turbidity was higher in hydrogels with compositions close to the critical point. For instance, hydrogels with 2% nPVA and 2.5% DS showed a significantly higher level of turbidity compared to 5% nPVA gels with the same DS concentration (Fig. 3F). Additionally, the turbidity of formulations with 4% nPVA and varying DS before and after UV curing was measured. Before UV curing, there is no significant difference in the turbidity. After UV curing, the turbidity of hydrogels with 1.5% DS was significantly higher compared to 0.5% DS and 1% DS groups as well as all groups before UV curing (Fig. 3G). This indicates that the composition with 4% nPVA and 1.5% DS is initially miscible, but phase separates upon polymerization.

**Table 1: Composition and properties of common mixes used in this study.** [a] Weight concentration was used to correlate with the approximated phase diagram; [b] determined by RIFFT of confocal imaging data, without/with swelling; [c] 5% gelatin was added to enhance the viscosity of the resin; [d] no pores detected; [e] before incubation at 37 °C (prior to gelatin release). DS (MW: 40 kDa), 0.05% LAP, PEG-2-SH crosslinker for a thiol:ene ratio at 4:5.

| Name | nPVA % a (w/w) | DS % (w/w) | Pore sizes [b] [μm] | G' [Pa] | G" [Pa] | G"/G' |
|---|---|---|---|---|---|---|
| Mix 0 | 2.0 | - | d NA | 220 ± 24 | 1.42 ± 0.34 | 0.007 ± 0.002 |
| Mix 1 | 2.0 | 2.5 | 8/3 | 370 ± 4 | 1.51 ± 0.09 | 0.004 ± 0.0002 |
| Mix 2 | 2.0 | 3.0 | 8/7 | N/A | N/A | N/A |
| Mix 3 | 2.0 | 3.1 | 2-8 / N/A | N/A | N/A | N/A |
| Mix 4[c] | 2.0 | 1.0 | N/A / 18 | e 4249 ± 1063 | e 94.2 ± 38.9 | e 0.022 ± 0.004 |

*Example 3: High Light Intensity Leads to Smaller Pores and Accelerates Crosslinking*

Tuning the pore size by light intensity is an important criterion for the applicability of these phase-separating resins for light-based 3D printing. Such tuning has been shown in purely organic polymeric matrices in literature, but no previous studies have demonstrated tuning of porosity in a hydrogel system. Thus, the effect of light intensity (5 - 100 mW $cm^{-2}$) on PIPS and pore size distribution was investigated. As anticipated, imaging of gels cured at different light intensities showed that the length scale of the pores can be tuned by the UV light intensity (Fig. 4A). At lower light intensity, the pores were larger. FFT analysis confirmed the decrease of the characteristic length scale with the increase of light intensity. The length scale was significantly different between 10 and 100 mW $cm^{-2}$ (Fig. 4B).

Photorheometry showed that an increase in light intensity accelerates crosslinking (Fig. 4C-F). The evolution of G' with different UV light intensities is shown in Fig. 4C. For analyzing the crosslinking speed, a linear regression at the section where the $R^2$ exceeds 0.99 was made as described in literature. The slope, which indicates the crosslinking speed, was higher with increased light intensity (Fig. 4D). The delay time denotes the timepoint where the linear regression curve starts (Fig. 4E), whereas $T_{80\%}$ refers to the time to reach the 80% of the maximum G' within 5 min of UV curing. The results confirmed that photocrosslinking is faster at higher light intensities (Fig. 4F).

Together, these findings suggest that the light intensity can be used to tune the crosslinking dynamics and PIPS, which influences the length scale of the pores. Kimura et al. (Soft Matter 2013, 9 (35), 8428-8437) reported phase separation induced by photopolymerization in a mixture of polystyrene and methacrylate. By changing the light intensity, a variety of stationary morphologies were obtained. With higher light intensity, the morphology has less time to evolve before it is arrested and frozen at the onset of the gelation.

*Example 4: Cellular Behaviour in Phase-separating Hydrogels*

For studying cellular behavior in the phase-separating hydrogels, human mesenchymal stem cells (hMSC) were photoencapsulated in different 3D environments. The cells were mixed with the hydrogel precursors and cured under a UV lamp (Fig. 5A). Phase-separating compositions with 2.5 and 3% DS showed a significantly higher viability (>90%) compared to the control without DS (Fig. 5B-C). The 2.5% DS group even showed an excellent viability (>95%) on average.

The phase-separating compositions also showed a higher average cell area compared to the control. Cells in 3% DS gels showed a significantly higher cell area compared to the control without DS on day 13 (Fig. 5D). Further, the cell area significantly increased over time. Similarly, the images of the actin-nuclei-stained cells showed longer dendrites in the phase-separating gels (Fig. 5E). The dendrite length increases over time in the phase-separating compositions and seems to be higher in 3% DS than in 2.5% DS gels, which can be explained by the larger pore size of 3% DS gels. Dendrites penetrating the pores were also observed.

*Example 5: Volumetric Bioprinting with An Optimized Photoresin*

The phase-separating resins were combined with the Readily3D tomographic volumetric printer, which allows rapid construction of 3D living hydrogel constructs in a rotating glass vial within seconds (Fig. 6A). Initial printing attempts were unsuccessful due to insufficient viscosity of the resins. Without additional components, no successful polymerization was observed in a laser dose test. Instead of polymerized dots, streaks were found, which implies that the material was partially polymerized but sedimented. In volumetric printing, a viscous or physically crosslinked material is needed. Gelatin was added to the composition as a viscosity enhancer and also a complementary thermo-reversible network.

A resin containing 2% nPVA, 1% DS, and 5% gelatin (Mix 4, Table 1) showed pores on the desired length scale. With higher DS concentrations of 2% DS and above, the gels were not transferrable after washing, which may be explained

by premature phase separation of gelatin with DS, as gelatin and dextran have been found to phase separate in literature as described by Butler and Heppenstall-Butler, Biomacromolecules 2003, 4, 928-936; https://doi.org/10.1021/bm0340319. Unwanted phase separation was seen with 1.5% DS, while no phase separation was seen with 0.5% DS. With 1% DS, pores in the range of about 2-5 μm were observed.

Further, the composition showed temperature-dependent gelation and a viscosity higher than water. A temperature sweep from 37 °C to 8 °C showed a gelation point at around 20-22 °C (Fig. 7A). Above 22 °C, the complex viscosity was about 120 mPa·s (Fig. 7B), which is roughly 200 times higher than the viscosity of water and almost seven times higher than the composition without gelatin. Crosslinking the composition at 25 °C is indicated by a clear increase in G' and G" after UV irradiation for 60 s (Fig. 7C). A plateau in G' of about 7 kPa was reached within less than one minute. The elastic modulus G' is much higher compared to compositions without gelatin.

Laser dose tests were performed to determine a suitable laser dose range for the resin. An estimate of 140 mJ $cm^{-2}$ was made with the first dose test. Then, a second test with smaller intervals was performed and a threshold of 75 mJ $cm^{-2}$ was found. 3D objects could be printed with a laser dose in the range of 95 to 125 mJ $cm^{-2}$. The volumetric prints and laser dose tests were conducted above room temperature because the ink turned turbid at room temperature, which may affect light penetration and printability. The resin remained as a viscous liquid during printing. The turbidity is presumably due to phase separation between gelatin and other components such as DS, which may occur when gelatin is physically crosslinked at 4 °C. At 90 mJ $cm^{-2}$ and below, a turbid structure was visible towards the end of the print but disappeared when swirling the vial. This can be explained by PIPS: as the material is polymerizing, phase separation is initiated because of a decrease in entropy even before the gel point is reached. When swirling, the phase-separated liquid resin is mixed with unpolymerized resin, leading to a lower concentration of polymerized material.

Using volumetric printing, a vascular branch model was printed within 12 seconds. The model (Fig. 6B) had a height of 1.4 cm, vascular channels with dimensions of 0.7-1.4 mm and was printed with a laser dose of 95 mJ $cm^{-2}$. The turbid construct is depicted while printing (Fig. 6D). Channels were shown to be perfusable after leaching out the gelatin for two days at 37 °C and subsequent incubation in a FITC-dextran solution for 24 h (Fig. 6D). The printing time is 12 s (Fig. 6C). The prints with 95 mJ $cm^{-2}$ led to ultrasoft constructs that are hard to transfer, whereas prints at higher doses such as 125 mJ $cm^{-2}$ resulted in stiffer constructs.

Next, we investigated if the printed constructs are indeed macroporous. After one day incubation in a solution of FITC-dextran, confocal imaging data showed interconnected pores in the printed gel constructs (Fig. 6E). The RIFFT of this image showed a clear peak corresponding to characteristic length scale of around 18 μm close to the border (Fig. 6F) and 17 μm in the middle of the gel. The background signal was lower when imaging at the border of the construct after incubation in FITC-dextran at 4 °C for one day. This observation may be either because the dye can penetrate the PVA hydrogel if given more time to diffuse or because leaching out gelatin left behind pores in the gel, which could be permeated by FITC-dextran.

An initial experiment showed that the branch structure could be printed in the presence of live hMSCs. After bioprinting, the channels were perfusable by phenol red stained medium. The gel appeared to be macroporous. Cells appeared highly viable as evidenced by a Calcein-AM staining.

*Example 6: Two-Photon-Induced Phase Separation*

With two-photon polymerization, patterns on a smaller scale could be printed in combination with PIPS (see Fig. 9). Like in volumetric printing, gelatin as a viscosity enhancer was added to enable printability. A higher nPVA concentration of 3% was necessary for successful polymerization compared to UV polymerization since the probability of two-photon absorption is much lower than single-photon absorption. The incorporation of gelatin reduced the diffusion issues as initially observed in the low viscosity formulations without gelatin. Arbitrary microstructures could be obtained with a laser power in the range of 10-80% for 2.5% DS and 10-100% for 3% DS, respectively. Interconnected pores were seen inside the patterned structures. The pore size was in the range of 2-13 μm and 2-8 μm for 2% and 3% nPVA respectively, as determined by FFT analysis (N>15 for both images). Before two-photon patterning, no pores were observed, suggesting that phase separation is induced by two-photon-induced polymerization.

*Example 7: Volumetric Bioprinting of a Perfusable Cell-Laden Construct*

After establishing the printing process parameters, we evaluated the bio-printability of nPVA resins for printing perfusable complex constructs. To shed light on how matrix stiffness impacts cell behaviors, two groups of bio-resins were formulated: 1.5% nPVA and 3% nPVA for the soft and stiff group, respectively. In this experiment, a soft bio-resin consisting of 1.5% nPVA, PEG-2-SH and 5% gelatin was used to fabricate perfusable constructs. As shown in Fig. 10, a perfusable hMSC-laden construct was successfully printed from a branch model within 15 s. Interestingly, cells in the printed construct could self-organize into irregularly shaped multicellular clusters and distribute in close proximity to inner channels following 16 h after the printing. Live-cell confocal imaging data revealed that hMSCs formed smaller aggregates in

the smaller channels (C2 and C3) while bigger cell clusters with round shapes were observed in the C1 channel (Fig. 10 B-D).

Actin-nuclei staining was used to detail the morphology of cell clusters after 7 days of culture (Fig. 10, E-I). Cells display an elongated morphology in alignment with the channel orientation (Fig. 10, E-G). Furthermore, cells residing outside the channels spreaded and even formed intercellular connections (Fig. 10, H-I). We reason that cells surrounding the channels have more access to nutrients as well as more physical freedom to self-organize into cell clusters. In addition, these results imply that geometrical cues in 3D tissue culture play an important role in generating aligned multicellular systems.

*Example 8: Volumetric Tomographic Photofabrication of Hetero-cellular Pre-Vascularized Hydrogel*

In biofabrication, 3D construction of hetero-cellular environments will provide the means to better mimic the native tissue environments where paracrine signaling is crucial for cell-cell communication and tissue development. To test if the nPVA bio-resins can be used for 3D co-culture applications, we seeded human umbilical vein endothelial cells (HUVECs) into the hollow channels of a 3D printed hMSC-laden construct towards a pre-vascularized model. Before co-culture, the hMSC-laden hydrogel construct was osteogenically differentiated for 7 days. After being dispersed in a collagen type I hydrogel, HUVECs were perfused into the channels before 3D co-culture for another 7 days (Fig. 11A). Confocal images evidenced that cells migrated out of the supporting collagen hydrogel and formed an endothelial monolayer after 72 h (Fig. 11B).

*Example 9: Volumetric Tomographic Photopatterning of Biochemical Motifs Within Preformed Hydrogels.*

Finally, we explored if the tomographic volumetric printing technique can be utilized to photochemically immobilize molecule-of-interests within a preformed hydrogel for spatiotemporal control of cell growth during 3D culture. It is hypothesized that the residual norbornene groups in a nPVA hydrogel formed by an off-stoichiometric crosslinking are accessible for radical-mediated thiol-ene photo-conjugation by tomographic photopatterning. This patterning process is realized in 3D in three steps as shown in Fig. 12A. First, a centimeter-scale hydrogel was printed by volumetric printing. Subsequently, the construct was immersed in a reaction buffer containing thiolated fluorescein (0.85 mM) and photoinitiator LAP (1.7 mM). After volumetric printing and thorough washing, the dye was covalently immobilized within the prescribed regions. For example, a 3D free-form helix model (Fig. 12B) as reported by Wang et al. (Adv Funct Mater 2020, 28 (45), 1804107), was printed within 16.3 s. Given the short printing time, cell-friendly light wavelength (405 nm) and unrestricted 3D geometry, we envisage this volumetric photografting method may open up new avenues for 3D bioprinting of complex tissues where bioactive molecules (e.g., proteins, growth factors) can be immobilized inside a 3D hydrogel to guide tissue growth in a spatially and temporally controlled fashion.

*Example 10: An Adapted Phase-separating Resin for Volumetric Bioprinting.*

We developed an adapted phase-separating resin that consists of lower concentrations of gelatin (4%) and DS (0.2%, 0.5%) for volumetric bioprinting. As shown in Fig. 13A, phase contrast microscopy images suggest that phase separation occurred immediately after in situ photocrosslinking of the acellular gels formed by adapted resins with varying amounts of DS. By contrast, the control sample without DS appears homogeneous. We further applied the resin with 0.2% DS for volumetric bioprinting. Confocal microscopy imaging of the volumetrically bioprinted hydrogel construct shows that FITC-dextran tracers permeated the pore space (Fig. 13B). RIFFT of the image shows a peak corresponding to an average pore size at a length scale of 2.2 $\mu$m (Fig. 13C).

*Conclusion*

In conclusion, a novel phase-separating photoresin for light-based 3D bioprinting was developed. Further, the pore size could be fine-tuned by the light intensity. The newly developed phase-separating nPVA gels showed a high viability on day one and increased spreading over two weeks. First successes were shown with 2PP and volumetric bioprinting, where a cm-scale construct with macropores could be printed in only 12 s. To the best of our knowledge, this is the first report of PIPS for 3D printing of cell-compatible macroporous hydrogels.

The developed phase-separating resins could be applicable to several fields of research. Although volumetric printing is most suitable for printing complex structures of clinically relevant sizes, the developed bio-resin could also be used for a variety of other light-based 3D printing techniques such as digital light processing. Even the use in extrusion bioprinting is foreseeable, as gelatin is thermo-responsive. In the case of light-based 3D printing, different pore sizes may be digitally 3D-printed within one object by tuning the light intensity and PIPS. Accordingly, complex hierarchical structures with macropores could be generated in the presence of living cells, which could significantly advance the fields of

biofabrication and functional tissue engineering.

*Example 10: Material and Methods*

*Materials*

A final concentration of 0.05% LAP was used throughout all experiments with UV curing. In two-photon photopatterning, 2 mM P2CK was used instead. nPVA (47kDa, DoF 7% or 20%) was synthesized as described elsewhere (Qin et al, Adv Mater 2018, 30, 1705564). It was used at different concentrations and dissolved in a solution containing the photoinitiator by vortexing and 15 - 30 min of ultrasonication. PEG-2-SH (2 kDa or 3.4 kDa, LaysanBio) was used as the crosslinker at a concentration that results in a thiol/ene ratio of 0.8. It was dissolved just before mixing in PBS with a pH of 6.05 for better stability. DS (40 kDa, Carl Roth) was used for phase separation and was dissolved in PBS at 50 °C for one to two hours with vortexing every half hour. Dextran (40 kDa, Sigma-Aldrich) was dissolved similarly. For cellular experiments, a fibronectinderived peptide CGRGDSP (China Peptides) was added to promote cell attachment. All components were dissolved in PBS.

*Approximation of a Phase Diagram*

An approximation of a phase diagram for the nPVAwith DS using a "mini-titration setup" was made. In a small glass vial, about 1 mL of a 10% (w/v) nPVA solution was prepared. 200 µl from this solution was transferred to a new glass vial. While stirring this nPVA solution with a small magnetic stirrer (8 x 3 mm), the dextran (20%, w/v) or DS (50%, w/v) solution was added dropwise with less than 10 µl drops until the solution turned visibly turbid (without turbidity disappearing) as described in literature (see Capela et al., Chem Eng Educ. 2019 Spring; 53(2): 112-120). The tube containing the DS/dextran solution was weighed before and after titration. The same was done also with lower nPVA concentrations of 5% and 1% (w/v). The receiving nPVA vial was weighed before and after titration to minimize any error caused by the retention of dextran on the pipette tip. For the phase diagram, the concentrations were all calculated to w/w.

*Hydrogel Preparation*

Thorough mixing is essential for PIPS. In this work, it was ensured by pipetting up and down 20 times after the addition of each component. When the volume was large enough, e.g. in volumetric printing experiments, the composition was mixed until it turned visibly clear or homogenous. In some cases, additional vortexing for two to three seconds was performed. In case of bubbles and small sample volumes, the tube was centrifuged for a few seconds and then vortexed again. The crosslinker was added last. Detailed descriptions on the hydrogel preparations can be found in the chapters of each composition.

*General Remarks on Hydrogel Precursor Preparation*

The concentrations are mostly given in w/v%, where the volume contribution of the solid part is neglected. As an example, 1% w/v means 0.01 g/mL. In compositions related to a phase diagram, w/w% was used for nPVA, PEG dithiol (PEG-2-SH) and DS as well as dextran, where the density of water was assumed to be 1 g/mL. In some experiments, a "10%" DS stock solution diluted by volume from a 50% stock solution was used. However, the density of the 50% stock solution was shown to be around 1.25 g/mL, so that the concentration of the diluted "10%" stock solution and so also the final DS concentrations in the hydrogel precursor mixture were higher than anticipated. The slightly higher average density of the diluted 10% stock solution compared to a directly prepared 10% stock solution also hints at this. In this report, 2.5% DS and 3% DS with 2% nPVA refer to compositions prepared from the diluted 10% DS stock solution, which correspond to actual concentrations of around 3.125% and 3.75% DS respectively as calculated by the density measurements. Tubes containing stock solutions were additionally sealed with parafilm as initial reproducibility issues were observed with stock solutions older than a month, where the concentration was probably higher than in the beginning due to water evaporation.

*Approach to Finding New Compositions*

Finding new compositions capable of phase separation and suitable for light-based 3D printing and cell embedding was an iterative process. Generally, compositions were chosen according to the approximate phase diagram with nPVA and DS. They were mostly chosen to be in the miscible region below the binodal line and as close as possible to the critical point. As the nPVA concentration was increased from 2 to 3% for example, lower DS concentrations were needed. The suitable DS concentration was screened for with fixed other components.

Firstly, additional components and compositions with them were explored with rheometry on viscosity and

gelation. Secondly, the three (nPVA, DS, PEG-2-SH) or more components where mixed and cured in micromolds and investigated by confocal laser scanning microscopy (CLSM) to investigate the pore morphology and the images were analyzed to find the characteristic length scale of the pores. If the composition shows a homogenous pore morphology on the desired scale, it was further explored by 3D printing and cell embedding. If one of the checkpoints were not passed, the composition was adjusted further.

*nPVA-DS Phase-Separating Composition*

The composition for Mix 1 is described in detail in **Table 2.** The components were added in the order listed, where it was especially important that the crosslinker is added last. For cell embedding, the order of mixing was altered to minimize the time where cells are outside of their medium (Table 3).

**Table 2:** Components of the 2% nPVA 2.5% DS composition.

| Component | Diluted in | Concentration [w/w] | Relative volume [v/v] | Final concentration [w/v] |
|---|---|---|---|---|
| **nPVA (DoF =7%)** | 0.1 % LAP | 4% | 50% | 2% (0.05% LAP) |
| **DS** | PBS | "10%" | 25% | 2.5% |
| **PBS** | | 1x | 12.5% | |
| **PEG-2-SH (2 kDa)** | PBS | 15.34% | 12.5% | 1.9% (thiol/ene ratio: 0.8) |

Table 3: Components of the 2% nPVA 3% DS composition used for cell embedding.

| Component | Diluted in | Concentration [w/w] | Relative volume [v/v] | Final concentration [w/v] |
|---|---|---|---|---|
| **DS** | PBS | "10%" | 30% | 3% |
| **PBS** | | 1x | 8% | |
| **CGRGDSP** | PBS | 14.10% | 2% | 0.28% (10% of thiol groups) |
| **nPVA (DoF = 7%) with cells** | 0.1 % LAP | 4%<br>1 Mio/mL | 50% | 2% (0.05% LAP)<br>0.5 Mio/mL |
| **PEG-2-SH (2 kDa)** | PBS | 16.09% | 10% | 1.9% (thiol/ene ratio: 0.8) |

*Bio-resin Based on nPVA-DS-gelatin*

Gelatin was explored mainly because it gelates at lower temperatures, which makes it suitable to prevent sedimentation in volumetric printing. Gelatin was dissolved at 40 °C in the waterbath for two to three hours for a stock solution of 10% (w/v). The composition used for printing experiments is described in **Table 4.** The components were mixed in this order and were warmed to prevent gelation during mixing. Note that the PEG-2-SH was not warmed to keep it stable. Cells were suspended in the gelatin portion.

Table 4: Composition of the 2% nPVA 1% DS 5% gelatin bio-resin.

| Component | Diluted in | Concentration | Relative volume [v/v] | Final concentration [w/v] |
|---|---|---|---|---|
| **Gelatin** | PBS | 10% w/v | 50% | 5% |
| **nPVA (DoF = 20%)** | 0.25 % LAP | 10% w/w | 20% | 2% (0.05% LAP) |
| **PBS** | | 1x | 10% | |
| **DS** | PBS | 10% w/w | 10% | 1% |
| **PEG-2-SH (2 kDa)** | PBS | 16.09% w/w | 10% | 1.9% |

*Rheometry*

For rheometry measurements, a modular Anton Paar MCR 302 photo-rheometer was used. A glass plate and parallel plate with 20 mm diameter (PP20) were used. The gap was set to 0.1 mm and 50 µl of the resin was loaded unless otherwise mentioned. To prevent drying, a wet tissue was placed within the temperature chamber. Mineral oil was loaded

surrounding the sample to prevent drying issues in longer measurements. For measuring crosslinking, time sweeps were performed. For photocrosslinking, a UV-LED lamp from Thorlabs at 365 nm was used. After one minute of measurement without light, the lamp was turned on at a power level of 68.7%, which corresponds to 20 mW $cm^{-2}$ in all measurements unless otherwise noted. For optimal results, the normal force was kept at zero to compensate for shrinking, which may occur during curing and lead to a negative normal force. Like this, the gel does not lose contact with the plate. Data points were collected each 5-10 s. A shear strain of 0.5% or 1% (for the temperature sweeps) and frequency of 1 Hz or angular frequency of 10 rad/s were set. UV curing was done at 22 °C for most experiments, or at 4 °C for resins with gelatin (no dextran sulfate), or at 25 °C for mimicking volumetric printing of phase-separating resins with gelatin and dextran sulfate. For temperature sweeps, the plate was cooled down at a rate of 1 °C per minute from 37 °C to 4 °C.

Frequency and amplitude sweeps were performed to assess the mechanical behavior of the hydrogels after curing. Right after the UV crosslinking measurement, the frequency sweep was done *in situ* with an angular frequency of 0.1 - 100 rad/s and a strain of 0.5%. In some cases, a subsequent amplitude sweep was performed with an angular frequency of 10 rad/s and a strain of 0.01 - 100% and in some cases even up to 1000%. For the previously cured and swollen hydrogels, the PP10 was used instead, and the gap thickness was set until reaching a normal force of around 0.1 N.

*Gel Casting in Molds*

The gels were casted in PDMS molds with a thickness of 0.5 mm, outer diameter of 10 mm and three wells with a diameter of 3 mm, which were punched out with biopsy punches. The molds were firmly attached to a coverslip (30 mm Ø) cleaned with acetone and isopropanol subsequently in an ultrasonic bath for 2 min. The wells were then filled with 7 μl of precursor solution each. Sigmacote-treated coverslips (10 mm or 15 mm Ø) were prepared beforehand by cleaning with isopropanol, dropping Sigmacote on top and washing off the excess after drying under the fume hood. For experiments with larger volumes such as gel transferability tests a Teflon mold was filled with 33 μl and covered with a Sigmacote-treated glass slide. After curing, PBS was added to the edge of the glass to facilitate loosening of the glass slide. The hydrogels were then transferred with a plastic spatula to a 12-well or 24-well plate filled with 1-2 mL of PBS for swelling. The dishes were sealed with parafilm and stored at 4 °C.

For two-photon polymerization, two layers of adhesive tape were used as the mold (thickness of 200 μm) and 3 mm diameter wells were punched out. Because of the reduced thickness, only 3 μl was needed per mold. For covalent fixation of the patterned structures, the coverslips were functionalized with sulfhydryl groups as follows: after cleaning with a tissue with isopropanol, the coverslips were submerged into 75 mL of ethanol solution with 12 drops of 3-mercaptopropyl) trimethoxysilane (Sigma-Aldrich) overnight. Then, they were washed twice in a water bath in ultrapure water (UPW) and dried with pressurized air and baked at 80 °C on filter paper within a petri dish for one hour. These coverslips were used for two-photon photopatterning of hydrogels.

*UV Curing*

The gels were cured for 5 min under UV lamps with an intensity of 10 - 20 mW $cm^{-2}$. The LAMAG UV lamp (365 nm) was used for most acellular experiments, the Navanino UV lamp (365 and 405 nm) for most cell embedding experiments and the Thorlabs UV lamp (365 nm) for fine tuning the light intensity. After curing, the sample was directly imaged. Washed samples were imaged after at least 4 days incubation in PBS at 4 °C. For the intensity tuning experiment, the gels with the light intensities 5 and 10 mW $cm^{-2}$ were cured for 20 min and 10 min respectively to ensure complete crosslinking. A power meter (S120VC, Thorlabs) with a sensor (PM100D S12VC, Thorlabs) was used to measure the light intensities.

*Permeability of Macroporous Hydrogels with FITC-Dextran*

The permeability of gels with FITC-dextran (500 kDa, Sigma-Aldrich) was studied as described elsewhere. In short, the gels were washed for three days at 4 °C in PBS till reaching a swelling equilibrium. Then, PBS was replaced with a solution containing 1 mg/ml FITC-dextran and the sample was imaged after incubation for 48 h.

*Turbidity Measurements*

The turbidity was measured as the absorption at 405 nm with a plate reader (Spark M10, Tecan). Before curing, 40 μl of the mixed precursor solution was loaded into a well of a 96-well plate. Gels cured in Teflon molds were punched with a 5 mm biopsy punch to fit into the plate and the turbidity was measured with and without additional 100 μl PBS loaded into the well. Multiple well reads (N=5) were used as replicates with a border of 2 mm.

*hMSC Cell Culture*

Frozen cells were thawed at 37 °C and suspended in Dulbecco's Modified Eagle's medium (DMEM). While centrifuging the cells at 300g for 10 min at 4 °C, 90 mL expansion medium (DMEM with 10% fetal bovine serum (FBS), 1% antibiotic-antimycotic (Anti-Anti), 1% non-essential amino acids and 0.001% bFGF aliquot) was loaded per triple flask. 10 mL of the cell suspension was then added to the flask. Incubation was done at 37 °C with 5% $CO_2$. On day 3 (d3), 50 mL expansion medium was added and on d5, the medium was changed with 100 mL per flask.

Cell splitting was performed when becoming 80-90% confluent. First, the cells were washed twice with PBS at 37 °C. Then, 15 mL of 0.05% trypsin-EDTA was loaded. After incubation at 37 °C for 4 min and tapping the flask to detach all cells, 15 mL control medium (DMEM with 10% FBS and 1% Anti-Anti) was added per flask and the cell suspension transferred to a falcon tube. After an additional washing step with 15 mL control medium, the cells were again centrifuged at 200g for 10 min at 4 °C. The cells were then resuspended in 10 mL control medium at 4 °C and counted using a hemocytometer and trypan blue. Finally, the cells were resuspended at the desired concentration after another centrifugation step in expansion medium and loaded into a fresh cell culture flask. Medium was changed three times a week thereafter and the cells again split once confluence was reached.

3D Cell Embedding

For cell embedding, expanding cells were collected as described above and resuspended in one of the hydrogel precursor components. The cell-laden precursor solutions were then transferred to casting molds. Using a UV365nm LED lamp (Thorlabs), the samples were UV cured for 5 min at an intensity of 10 mW $cm^{-2}$. The precursor materials were mostly sterilized either by filtering (PEG-2-SH, dextran, DS, peptides, LAP), weighing with sterile tools into sterile tubes and dissolved in a laminar flow hood with sterile PBS. Tools were sterilized by autoclaving mostly.

*Live/Dead Assay*

After washing with warm PBS, a staining solution containing 4 μl Calcein AM and 2 μl ethidium homodimer-1 (EthD-1) was loaded. The sample was then incubated for 15 min and washed twice with PBS. With the sample in PBS, the dish was sealed with parafilm and imaged within one hour. After that, PBS was replaced with osteogenic medium (control medium, 50 μg/ml ascorbic acid, 100 nM dexamethasone, 10 mM beta-glycerophosphate).

*Fixation and Actin-Nuclei Staining*

The samples were fixed with 4% PFA by washing with warm PBS, incubating with 4% PFA for 15 min at RT and washing twice with PBS. PBS was then added for hydration and the sample stored at 4 °C until the staining procedure was started. For the staining, the sample was first blocked with 1% BSA for 1 h and then permeabilized in 0.2% Triton X-100. After washing thrice with PBS, a staining solution was added, which contained 0.1% BSA, Phalloidin 647 and Hoechst (1 mg/mL) at dilutions of 1:200 and 1:1000 respectively. After 1-2 hours of incubation at RT protected from light, the sample was washed thrice with PBS and PBS was loaded for hydration.

*Volumetric (Bio)-Printing*

The Readily3D volumetric printer was used with the Apparite software. First, a laser dose test was conducted, in which defined spots were irradiated with varying laser intensity (405 nm) and irradiation time. For this, 500 μl of precursor solution were loaded into a quartz cuvette (Thorlabs). The visible dots were noted in a table after the irradiation was done. A second test with a smaller range could then be conducted to more accurately determine the threshold needed for polymerization. For the print, commonly 3-4 mL of precursor solution was prepared and loaded into 18 mm diameter Pyrex vials. The refractive index of GelMA (1.37) was chosen and the peak-to-average ratio was set to 6:1. The optimal settings as calculated by the software were used.

For bioprinting, the components were mixed in sterile conditions and loaded into an autoclaved vial. The vial was then cooled for 5 min in the 4 °C fridge to prevent cell sedimentation during transfer. Then, the vial was directly brought to the printer under light protection and subsequently printed. For some of the resins, phase separation occurred due to incubation at low temperature of 4 °C. The vials were warmed to room temperature in 15 min before the bioprinting. The laser dose found suitable for acellular printing was used. After visually confirming that a structure was printed, the closed vial was brought back to the laminar flow hood, transferred to an Ibidi glass bottom dish and medium was added.

*Volumetric Photopatterning*

A 3% nPVA resin was used to print a cylindrical hydrogel construct and washed in PBS at 37 °C for 3 days. Then the printed construct was immersed in a solution containing 0.85 mM thiolated FITC dye and 1.7 mM LAP overnight at 4 °C. To reduce disulfide formation during photopatterning, tris(2-carboxyethyl)phosphine (TCEP, 2 mM) was added to the solution. The stained hydrogel construct was transferred to a glass vial (diameter 10 mm) to perform volumetric photopatterning on the Tomolite printer followed by a thorough washing step with several changes of PBS for two days to remove non-reacted dye. Finally, the patterned objects were imaged with a stereomicroscope (Leica M205 FA) coupled with a DFC550 CCD color camera equipped with X-Cite 200DC lamp with a 3 mm light guide and widefield epifluorescence filter.

*Two-Photon Polymerization*

For two-photon patterning, the multiphoton microscope Leica SP8 MP was used with a 25x water objective and the MP laser MaiTai at 780 nm. The laser parameters were screened as follows: The offset was adjusted to 51.9 for only irradiating the region of interest (ROI). The array pattern was defined using the tilescan or "mark experiment" function with an offset of 250 μm in x/y. After moving to the first location, the tilescan function was turned off and the patterning performed. A square ROI with 100 μm long sides was drawn, a 50 μm thick z-stack starting at least 5 μm below the glass interface to ensure attachment was defined, and the z-step size was set to 1. For the patterning, the 3D ROI was chosen for the MP. Then, the tilescan function was used to move to the second position, where one of the laser parameters was changed. Images were taken using the 488 nm laser.

*Imaging and Image Analysis*

The confocal microscopes Leica SP8 MP and Leica SP8 AOBS CARS were used for imaging. For imaging the whole volumetric print, a stereomicroscope (Leica Stereo) was used. All microscopes were used with the software LASX. Images and videos during the volumetric printing process were captured with the integrated camera of the volumetric printer. Other images were taken with a smartphone. Image analysis was mostly performed in Fiji and is described in the following sections.

*2D Fast Fourier Transform*

2D-FFT is a common way to characterize the length scale of pores in bi-continuous structures. The analysis was done with a MATLAB code, which can be found in the supplementary information. In short, an image is read and converted to greyscale. Then the 2D-FFT of the image multiplied by a hamming window is taken. Further, radial binning is performed to show the radial intensity of the FFT (RIFFT). The length scale was then calculated as 1/q.

*Porosity Analysis*

Confocal z-stacks of 20-30 μm were obtained and deconvoluted using Huygens Professional Software. These images were processed with a MATLAB algorithm developed by Vandaele et al. (Soft Matter, 2020. 16(17): 4210-4219) that uses image segmentation and approximation of pores by spheres to quantify pore size and interconnectivity. Specifically, a binary image is generated after segmentation. Each pixel (2D) or voxel (3D) is classified as belonging to a pore (value 0) or to a hydrogel scaffold (value 1), depending on its intensity (below or above a threshold value). Then, the obtained binary volume is cleaned from small artefacts as a post-processing step. Pores with a volume smaller than 0.0065 $\mu m^3$ (diameter of approx. 0.12 μm) was removed from the analysis. An adaptive threshold sensitivity of 0.6 was chosen and pixel size was set to 72.2 nm. Pore size (external pore diameter in 3D) and pore connectivity (number of neighboring pores) were obtained for each group.

*Cell Viability and Cell Area Quantification*

Maximum intensity projections (MIPs) of 3x100 μm thick z-stacks were analyzed per group. Live cells were counted manually because the cells were not clearly separated. For the cell area quantification, the threshold was manually set and kept the same for all groups within one experiment. The area was measured by the actin signal. The cell nuclei were then manually counted. The average cell area was then calculated by the total area divided by the cell number for each z-stack.

*Statistical Analysis*

Statistical analysis was performed in GraphPad Prism 8.2.0, except for the slope analysis, which was performed in Excel. Ordinary one-way or two-way analysis of variance (ANOVA) were performed depending on the number of variables. They were followed by Sidak's or Tukey's test for multiple comparisons for two or more groups respectively. If the standard deviations did not appear equal among groups, the Brown-Forsynthe and Welch ANOVA test was followed by Dunnett's multiple comparison test was applied. In the two-way ANOVA, only simple effects within rows and columns analyzed. Welch's t-test was used when there were only two groups. The applied test is noted for each result. P values less than 0.05 were considered significant and are mentioned in the figure captions.

## Claims

1. A composition comprising:

    a. a first polymer susceptible to photo-crosslinking, a photoinitiator and optionally, a crosslinking agent, wherein the first polymer is functionalized with double-bond-containing moieties selected from norbornene, acrylate, vinyl ester, vinyl ether, vinyl carbonate, allyl ether, methacrylate, vinyl sulfone, and maleimide; and
    b. a second polymer not susceptible to photo-crosslinking, wherein the second polymer is a sulfated or non-sulfated polysaccharide;

    in aqueous solution,
    wherein the first polymer and the second polymer are miscible when the first polymer is not crosslinked, and wherein the mixture comprised of the first polymer and the second polymer undergoes phase separation, separating said first and said second polymer into separate phases, if the first polymer is crosslinked.

2. The composition according to claim 1, wherein the first polymer is a norbornene-functionalized polyvinyl alcohol, and a dithiol crosslinking agent is present in the composition.

3. The composition according to claim 2, wherein the norbornene-functionalized polyvinyl alcohol is a polyvinylalcohol-cis-5-norbornene-2,3-dicarboxylic acid ester.

4. The composition according to claim 2 or 3, wherein the norbornene-functionalized polyvinyl alcohol has a molecular mass of 3 kDa - 1000 kDa, particularly of 5 kDa - 200 kDa, more particularly in the range of 10 kDa - 80 kDa.

5. The composition according to any one of claims 2 to 4, wherein the norbornene-functionalized polyvinyl alcohol has one norbornene moiety per 5 to 10 free hydroxy moieties.

6. The composition according to any one of the preceding claims 1 to 5, wherein the second polymer is a sulfated or non-sulfated polysaccharide, particularly a sulfated polysaccharide.

7. The composition according to claim 6, wherein the polysaccharide is selected from the group comprised of dextran sulfate, chondroitin sulfate, sulfated alginate, particularly wherein the polysaccharide is selected from the group comprised of dextran sulfate and chondroitin sulfate, more particularly wherein the polysaccharide is dextran sulfate.

8. The composition according to any one of the preceding claims 1 to 7, wherein the concentration of the **first polymer** is 0.5-50% (m/m), particularly 0.5-10% (m/m), more particularly 2-3% (m/m);

    particularly wherein a crosslinking agent is present and the concentration of the crosslinking agent is 0.5-3% (m/m),
    particularly wherein the concentration of the crosslinking agent is 1 to 3% m/m;
    more particularly wherein the crosslinking agent is a thiol-functionalized PVA.

9. The composition according to any one of the preceding claims 1 to 8, wherein the composition comprises a first polymer comprising reactive ene (double bond) moieties, and the composition further comprises a crosslinking agent comprising thiol moieties, and the stoichiometric ratio of thiol moieties in the crosslinking agent to ene moieties in the first polymer is selected to range from 1:10 to 2:1, particularly the thiol:ene ratio ranges from 1:5 to 2:1, even more

particularly, from 2:5 to 6:5.

**10.** The composition according to any one of the preceding claims 1 to 9, wherein the concentration of the **second polymer** is 0.1% to 10% (m/m),
particularly wherein the concentration of the second polymer is 0.2% to 4.5% (m/m).

**11.** The composition according to any one of the preceding claims 1 to 10, further comprising a viscosity enhancer, particularly wherein the viscosity enhancer is selected from the group comprising gelatin, hyaluronic acid, gellan gum, poloxamers (block copolymers of ethylene oxide and propylene oxide, marketed as Pluronic®).

**12.** A method to generate a hydrogel object, particularly an implant, comprising the steps of:

a. providing a composition according to any one of the preceding claims 1 to 11;
b. sequentially illuminating different adjacent portions of the composition with light to provide the hydrogel object.

**13.** A hydrogel object, particularly an implant, obtained by the method according claim 12.

**14.** The hydrogel object according to claim 13, wherein the hydrogel is **characterized by**:

a. the presence of pores, wherein the pores are of a size of 0.2-1000 μm, particularly 500 nm - 100 μm;
b. interconnectivity of 20-100%, particularly of 40-100%;
c. porosity of 10-95%, particularly 40-90%;
d. a stiffness of 1-1000000 Pa, particularly 10-10000 Pa, more particularly 10-5000 Pa, even more particularly 20-1000 Pa;
e. viscoelasticity, wherein the half-time for stress-relaxation ($t_{1/2}$) is in the range of 5-10000 s, more particularly 10-2000 s, even more particularly 20-500 s.

**15.** The hydrogel object according to any one of claims 13 or 14, further comprising live mammalian cells, particularly human mesenchymal stem cells (hMSC) and/or human umbilical vein endothelial cells (HUVEC).

**Patentansprüche**

**1.** Eine Zusammensetzung, umfassend:

a. ein erstes Polymer, das zur photochemischen Vernetzung neigt, einen Photoinitiator und optional ein Vernetzungsmittel,
wobei das erste Polymer mit Doppelbindungen enthaltenden funktionellen Gruppen funktionalisiert ist, die aus Norbornen, Acrylat, Vinylester, Vinylether, Vinylcarbonat, Allylether, Methacrylat, Vinylsulfon und Maleimid ausgewählt sind;
und
b. ein zweites Polymer, das nicht zur photochemischen Vernetzung neigt, wobei das zweite Polymer ein sulfatiertes oder nicht sulfatiertes Polysaccharid ist;

in wässriger Lösung,
wobei das erste Polymer und das zweite Polymer mischbar sind, wenn das erste Polymer nicht vernetzt ist, und wobei die Mischung aus dem ersten Polymer und dem zweiten Polymer eine Phasentrennung durchläuft, bei der das erste und das zweite Polymer in getrennte Phasen separiert werden, wenn das erste Polymer vernetzt ist.

**2.** Die Zusammensetzung nach Anspruch 1, wobei das erste Polymer ein mit Norbornen funktionalisierter Polyvinylalkohol ist und ein Dithiol-Vernetzungsmittel in der Zusammensetzung vorhanden ist.

**3.** Die Zusammensetzung nach Anspruch 2, wobei der mit Norbornen funktionalisierte Polyvinylalkohol ein Polyvinylalkohol-cis-5-Norbornen-2,3-Dicarbonsäureester ist.

**4.** Die Zusammensetzung nach Anspruch 2 oder 3, wobei der mit Norbornen funktionalisierte Polyvinylalkohol eine Molekülmasse von 3 kDa bis 1000 kDa, insbesondere von 5 kDa bis 200 kDa, insbesondere im Bereich von 10 kDa bis 80 kDa aufweist.

5. Die Zusammensetzung nach einem der Ansprüche 2 bis 4, wobei der mit Norbornen funktionalisierte Polyvinylalkohol eine Norbornen-Einheit pro 5 bis 10 freie Hydroxylgruppen aufweist.

6. Die Zusammensetzung nach einem der vorstehenden Ansprüche 1 bis 5, wobei das zweite Polymer ein sulfatiertes Polysaccharid ist.

7. Die Zusammensetzung nach Anspruch 6, wobei das Polysaccharid aus der Gruppe umfassend Dextransulfat, Chondroitinsulfat und sulfatiertem Alginat ausgewählt ist, insbesondere wobei das Polysaccharid aus der Gruppe umfassend Dextransulfat und Chondroitinsulfat ausgewählt ist, und insbesondere wobei das Polysaccharid Dextransulfat ist.

8. Die Zusammensetzung nach einem der vorstehenden Ansprüche 1 bis 7, wobei die Konzentration des **ersten Polymers** 0,5-50 % (m/m), insbesondere 0,5-10 % (m/m), insbesondere 2-3 % (m/m) beträgt;

   insbesondere wobei ein Vernetzungsmittel vorhanden ist und die Konzentration des Vernetzungsmittels 0,5 bis 3 % (m/m) beträgt,
   insbesondere wobei die Konzentration des Vernetzungsmittels 1 bis 3 % (m/m) beträgt;
   insbesondere wobei das Vernetzungsmittel ein thiolfunktionalisiertes PVA ist.

9. Die Zusammensetzung nach einem der vorstehenden Ansprüche 1 bis 8, wobei die Zusammensetzung ein erstes Polymer umfasst, das reaktive En-Gruppen (Doppelbindungen) enthält, und die Zusammensetzung ferner ein Vernetzungsmittel umfasst, das Thiolgruppen enthält, und das stöchiometrische Verhältnis der Thiolgruppen im Vernetzungsmittel zu den En-Gruppen im ersten Polymer so gewählt ist, dass es im Bereich von 1:10 bis 2:1 liegt, insbesondere liegt das Thiol:En-Verhältnis im Bereich von 1:5 bis 2:1, insbesondere von 2:5 bis 6:5.

10. Die Zusammensetzung nach einem der vorstehenden Ansprüche 1 bis 9, wobei die Konzentration des **zweiten Polymers** 0,1 % bis 10 % (m/m) beträgt, insbesondere wobei die Konzentration des zweiten Polymers 0,2 % bis 4,5 % (m/m) beträgt.

11. Die Zusammensetzung nach einem der vorstehenden Ansprüche 1 bis 10, die ferner einen Verdickungsmittel umfasst, insbesondere wobei das Verdickungsmittel aus der Gruppe umfassend Gelatine, Hyaluronsäure, Gellangummi und Poloxamere (Blockcopolymere aus Ethylenoxid und Propylenoxid, im Handel als Pluronic® erhältlich) ausgewählt ist.

12. Verfahren zur Herstellung eines Hydrogelobjekts, insbesondere eines Implantats, umfassend die folgenden Schritte:

   a. bereitstellen einer Zusammensetzung gemäß einem der vorstehenden Ansprüche 1 bis 11;
   b. nacheinander bestrahlen von verschiedenen benachbarten Bereichen der Zusammensetzung mit Licht, um das Hydrogelobjekt zu bilden.

13. Ein Hydrogelobjekt, insbesondere ein Implantat, das nach dem Verfahren gemäß Anspruch 12 hergestellt wurde.

14. Das Hydrogelobjekt nach Anspruch 13, wobei das Hydrogel durch Folgendes gekennzeichnet ist:

   a. das Vorhandensein von Poren, wobei die Poren eine Größe von 0,2-1000 μm, insbesondere von 500 nm bis 100 μm, aufweisen;
   b. Vernetzbarkeit von 20-100 %, insbesondere von 40-100 %;
   c. Porosität von 10-95 %, insbesondere 40-90 %;
   d. eine Festigkeit von 1-1000000 Pa, insbesondere 10-10000 Pa, insbesondere 10-5000 Pa, und insbesondere 20-1000 Pa;
   e. Viskoelastizität, wobei die Halbwertszeit für die Spannungsrelaxation ($t_{1/2}$) im Bereich von 5 bis 10000 s, insbesondere von 10 bis 2000 s, und insbesondere von 20 bis 500 s liegt.

15. Das Hydrogelobjekt nach einem der Ansprüche 13 oder 14, das ferner lebende Säugetierzellen, insbesondere menschliche mesenchymale Stammzellen (hMSC) und/oder menschliche Nabelschnurvenen-Endothelzellen (HUVEC), umfasst.

## Revendications

**1.** Composition comprenant :

a. un premier polymère susceptible de photoréticulation, un photoinitiateur et en option, un agent de réticulation, dans laquelle le premier polymère est fonctionnalisé avec des groupes caractéristiques contenant une double liaison sélectionnés parmi le norbornène, l'acrylate, l'ester de vinyle, l'éther de vinyle, le carbonate de vinyle, l'éther d'allyle, le méthacrylate, le vinylsulfone et le maléimide ;
et
b. un second polymère non susceptible de photoréticulation, dans laquelle le second polymère est un polysaccharide sulfaté ou non sulfaté ;

en solution aqueuse,
dans laquelle le premier polymère et le second polymère sont miscibles lorsque le premier polymère n'est pas réticulé, et dans laquelle le mélange constitué du premier polymère et du second polymère subit une séparation de phases, séparant ledit premier et ledit second polymère en phases séparées, si le premier polymère est réticulé.

**2.** Composition selon la revendication 1, dans laquelle le premier polymère est un alcool polyvinylique à fonction norbornène et un agent de réticulation au dithiol est présent dans la composition.

**3.** Composition selon la revendication 2, dans laquelle l'alcool polyvinylique à fonction norbornène est un ester d'acide alcool polyvinylique-cis-5-norbornène-2,3-dicarboxylique.

**4.** Composition selon la revendication 2 ou 3, dans laquelle l'alcool polyvinylique à fonction norbornène a une masse moléculaire de 3 kDa à 1000 kDa, en particulier 5 kDa à 200 kDa, plus particulièrement dans la plage de 10 kDa à 80 kDa.

**5.** Composition selon l'une quelconque des revendications 2 à 4, dans laquelle l'alcool polyvinylique à fonction norbornène a un groupe caractéristique de norbornène pour 5 à 10 groupes caractéristiques hydroxy libres.

**6.** Composition selon l'une quelconque des revendications précédentes 1 à 5, dans laquelle le second polymère est un polysaccharide sulfaté ou non sulfaté, en particulier un polysaccharide sulfaté.

**7.** Composition selon la revendication 6, dans laquelle le polysaccharide est sélectionné parmi le groupe constitué du sulfate de dextrane, du chondroïtine sulfate, de l'alginate sulfaté, en particulier dans laquelle le polysaccharide est sélectionné parmi le groupe constitué du sulfate de dextrane et du chondroïtine sulfate, plus particulièrement dans laquelle le polysaccharide est le sulfate de dextrane.

**8.** Composition selon l'une quelconque des revendications précédentes 1 à 7, dans laquelle la concentration en le **premier polymère** est de 0,5 à 50 % (m/m), en particulier 0,5 à 10 % (m/m), plus particulièrement 2 à 3 % (m/m) ;

en particulier dans laquelle un agent de réticulation est présent et la concentration en l'agent de réticulation est de 0,5 à 3 % (m/m),
en particulier dans laquelle la concentration en l'agent de réticulation est de 1 à 3 % m/m ;
plus particulièrement dans laquelle l'agent de réticulation est un PVA à fonction thiol.

**9.** Composition selon l'une quelconque des revendications précédentes 1 à 8, dans laquelle la composition comprend un premier polymère comprenant des groupes caractéristiques (à double liaison) à ène réactif, et la composition comprend en outre un agent de réticulation comprenant des groupes caractéristiques thiol, et le rapport stœchiométrique de groupes caractéristiques thiol dans l'agent de réticulation sur des groupes caractéristiques ène dans le premier polymère est sélectionné dans la plage allant de 1/10 à 2/1, en particulier le rapport thiol/ène va de 1/5 à 2/1, encore plus particulièrement des modes de réalisation, de 2/5 à 6/5.

**10.** Composition selon l'une quelconque des revendications précédentes 1 à 9, dans laquelle la concentration en le **second polymère** est de 0,1 % à 10 % (m/m),
en particulier dans laquelle la concentration en le second polymère est de 0,2 % à 4,5 % (m/m).

**11.** Composition selon l'une quelconque des revendications précédentes 1 à 10, comprenant en outre un amplificateur de

viscosité, en particulier dans laquelle l'amplificateur de viscosité est sélectionné parmi le groupe comprenant de la gélatine, de l'acide hyaluronique, de la gomme gellane, des poloxamères (copolymères à blocs d'oxyde d'éthylène et d'oxyde de propylène, commercialisés en tant que Pluronic®).

**12.** Procédé de génération d'un objet en hydrogel, en particulier d'un implant, comprenant les étapes consistant à :

a. fournir une composition selon l'une quelconque des revendications précédentes 1 à 11 ;
b. éclairer séquentiellement différentes portions adjacentes de la composition avec de la lumière pour fournir l'objet en hydrogel.

**13.** Objet en hydrogel, en particulier implant, obtenu par le procédé selon la revendication 12.

**14.** Objet en hydrogel selon la revendication 13, dans lequel l'hydrogel est **caractérisé par** :

a. la présence de pores, dans lequel les pores sont d'une taille de 0,2 à 1000 μm, en particulier 500 nm à 100 μm ;
b. une interconnectivité de 20 à 100 %, en particulier de 40 à 100 % ;
c. une porosité de 10 à 95 %, en particulier 40 à 90 % ;
d. une rigidité de 1 à 1 000 000 Pa, en particulier 10 à 10 000 Pa, plus particulièrement 10 à 5000 Pa, encore plus particulièrement 20 à 1000 Pa ;
e. une viscoélasticité, dans lequel le demi-temps pour la relaxation en contrainte ($t_{1/2}$) est dans la plage de 5 à 10 000 s, plus particulièrement 10 à 2000 s, encore plus particulièrement 20 à 500 s.

**15.** Objet en hydrogel selon l'une quelconque des revendications 13 ou 14, comprenant en outre des cellules mammifères vivantes, en particulier des cellules souches mésenchymateuses humaines (hMSC) et/ou des cellules endothéliales de veine ombilicale humaine (HUVEC).

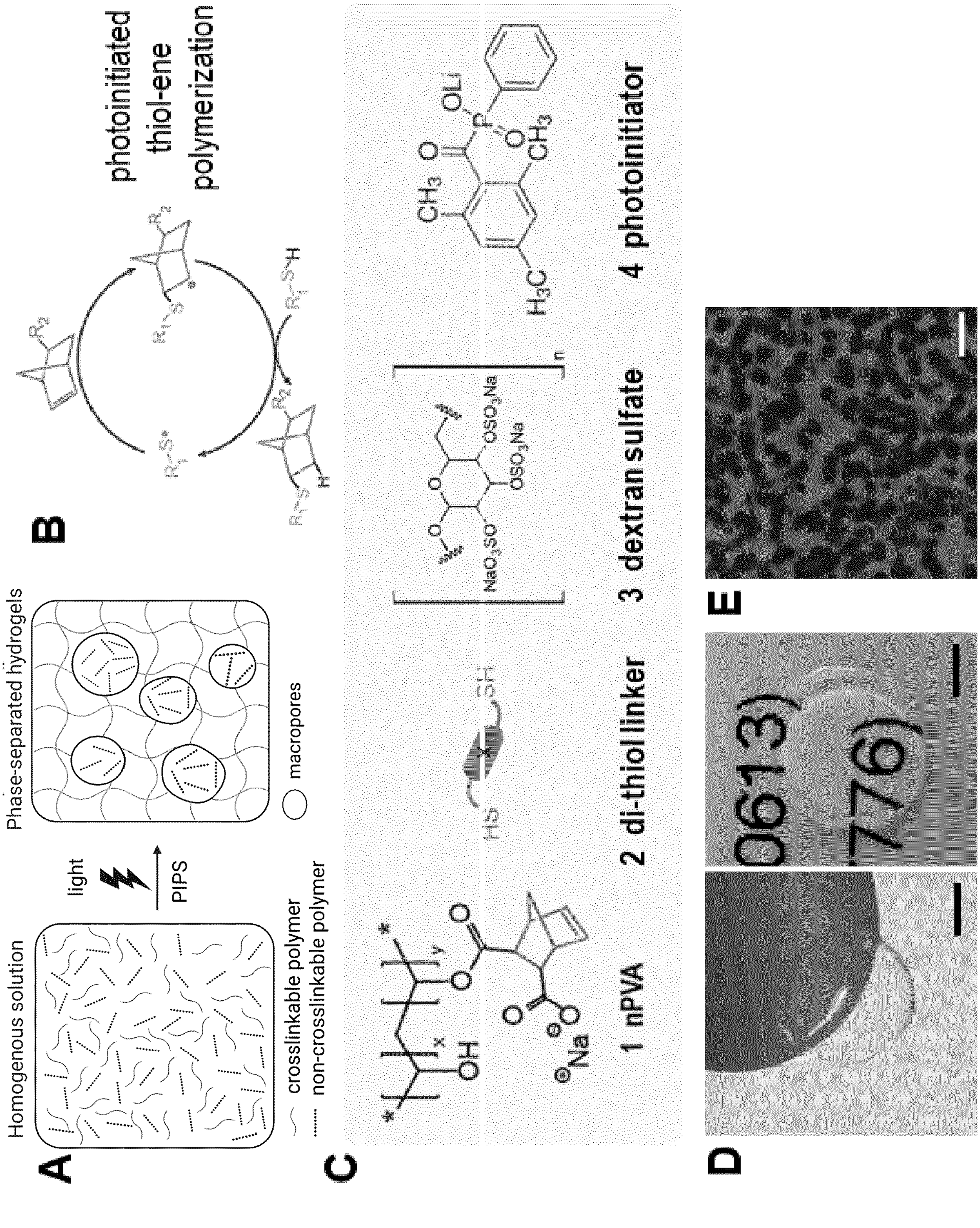
A
Homogenous solution
light
PIPS
Phase-separated hydrogels
crosslinkable polymer
non-crosslinkable polymer
macropores
B
photoinitiated thiol-ene polymerization
C
1 nPVA
2 di-thiol linker
3 dextran sulfate
4 photoinitiator
D
E

Fig. 1

Fig. 2

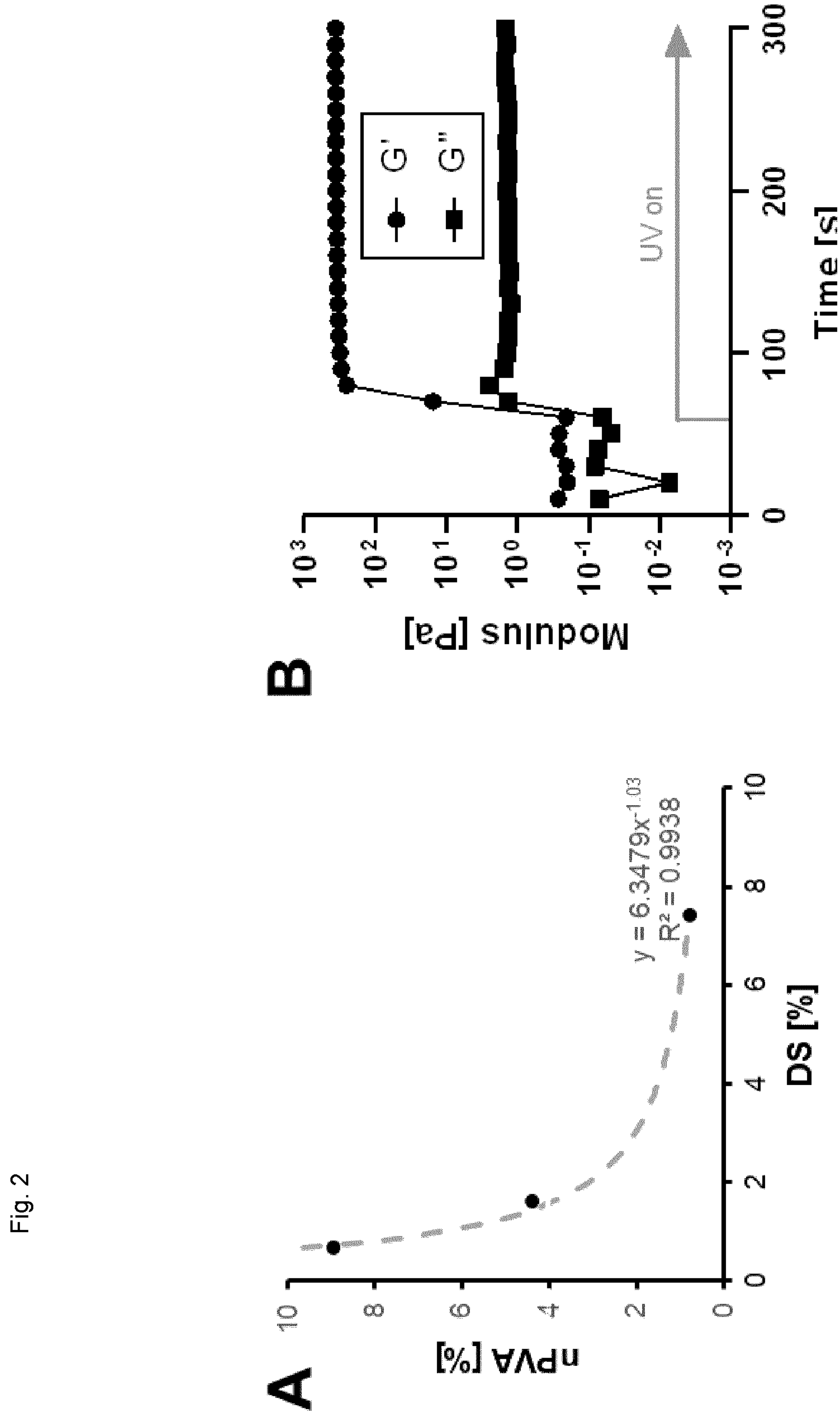

Fig. 2 (continued)

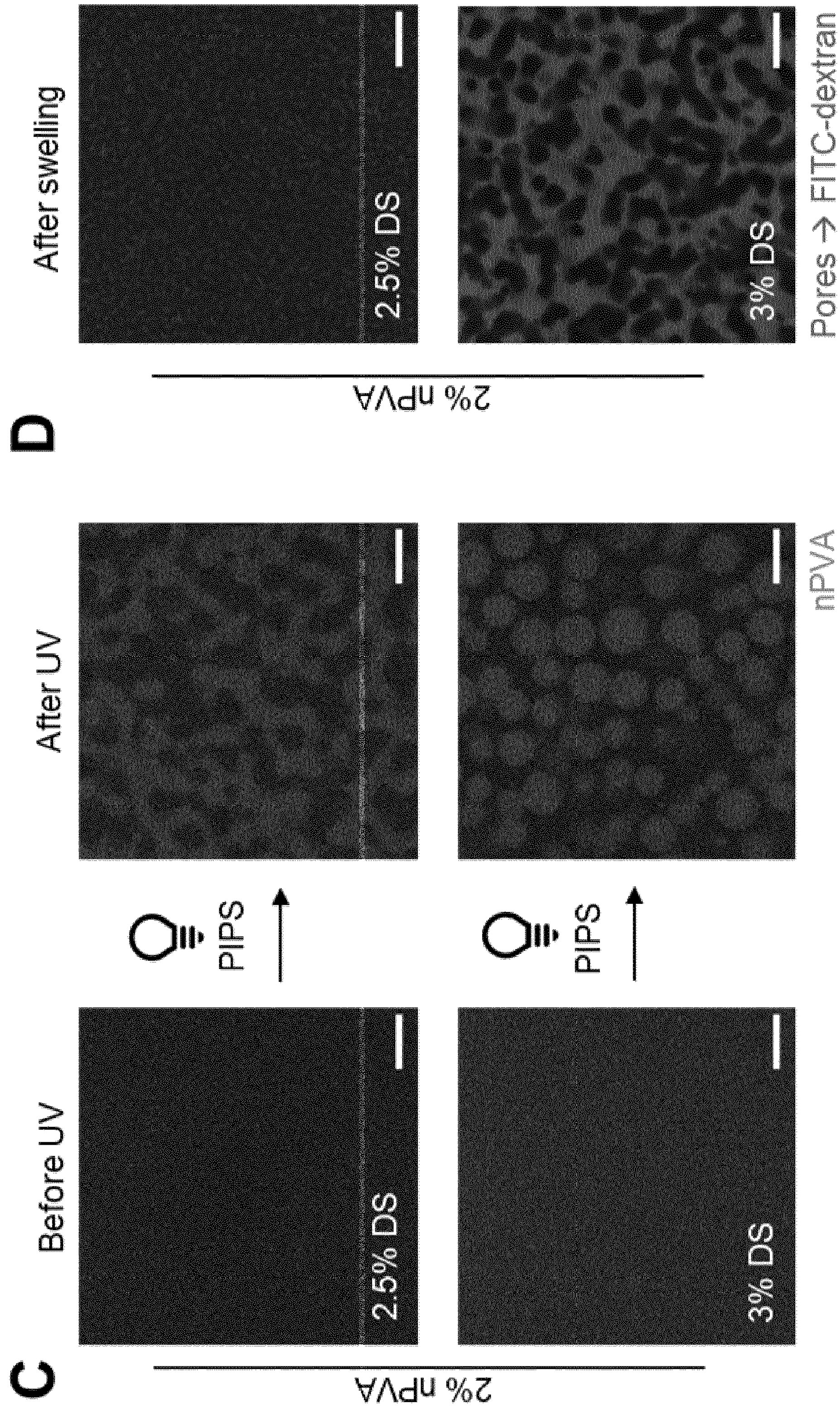

Fig. 3

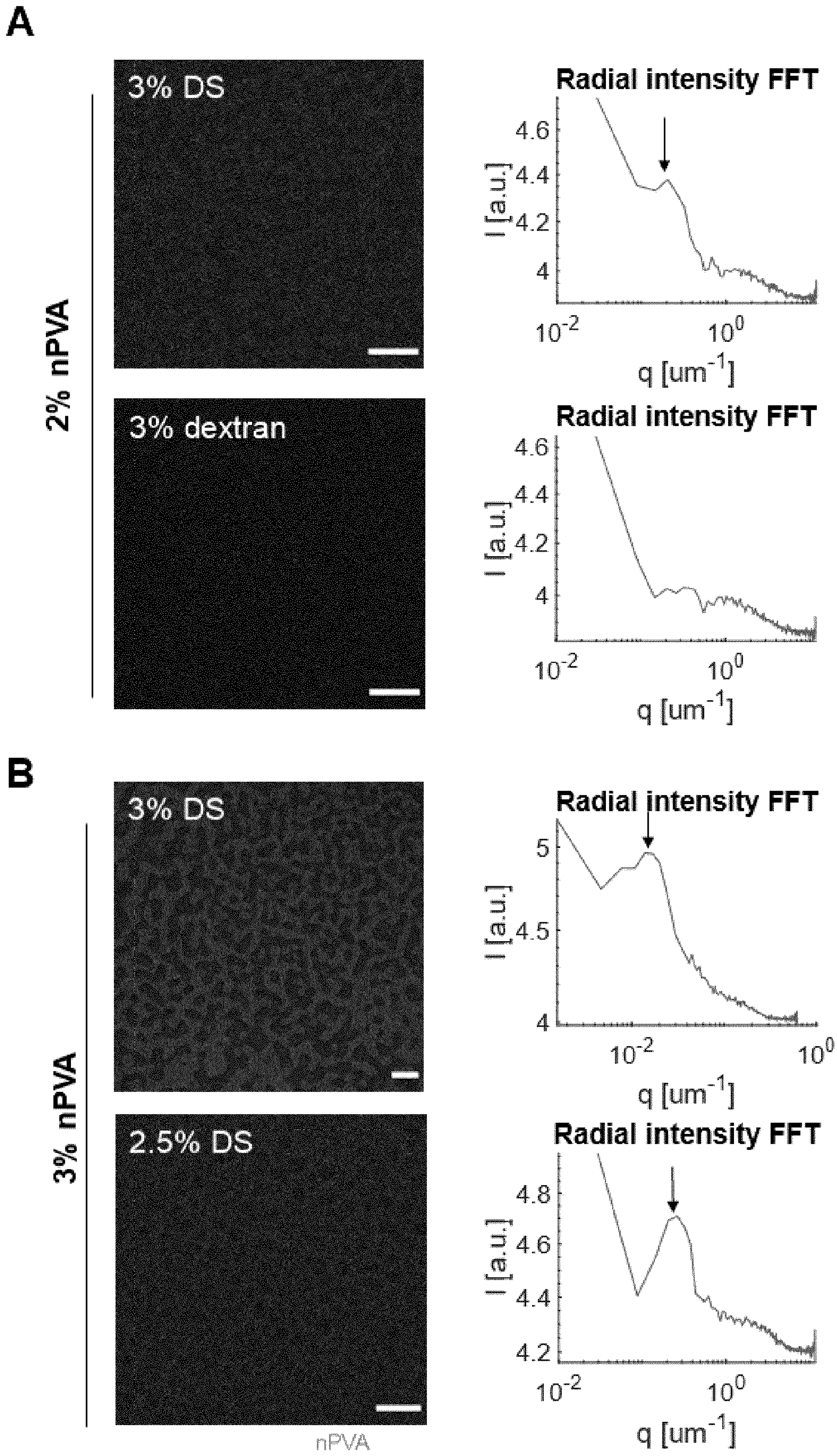
A
2% nPVA
3% DS
3% dextran
Radial intensity FFT
I [a.u.]
q [um-1]
B
3% nPVA
3% DS
2.5% DS
nPVA

Fig. 3 (continued)

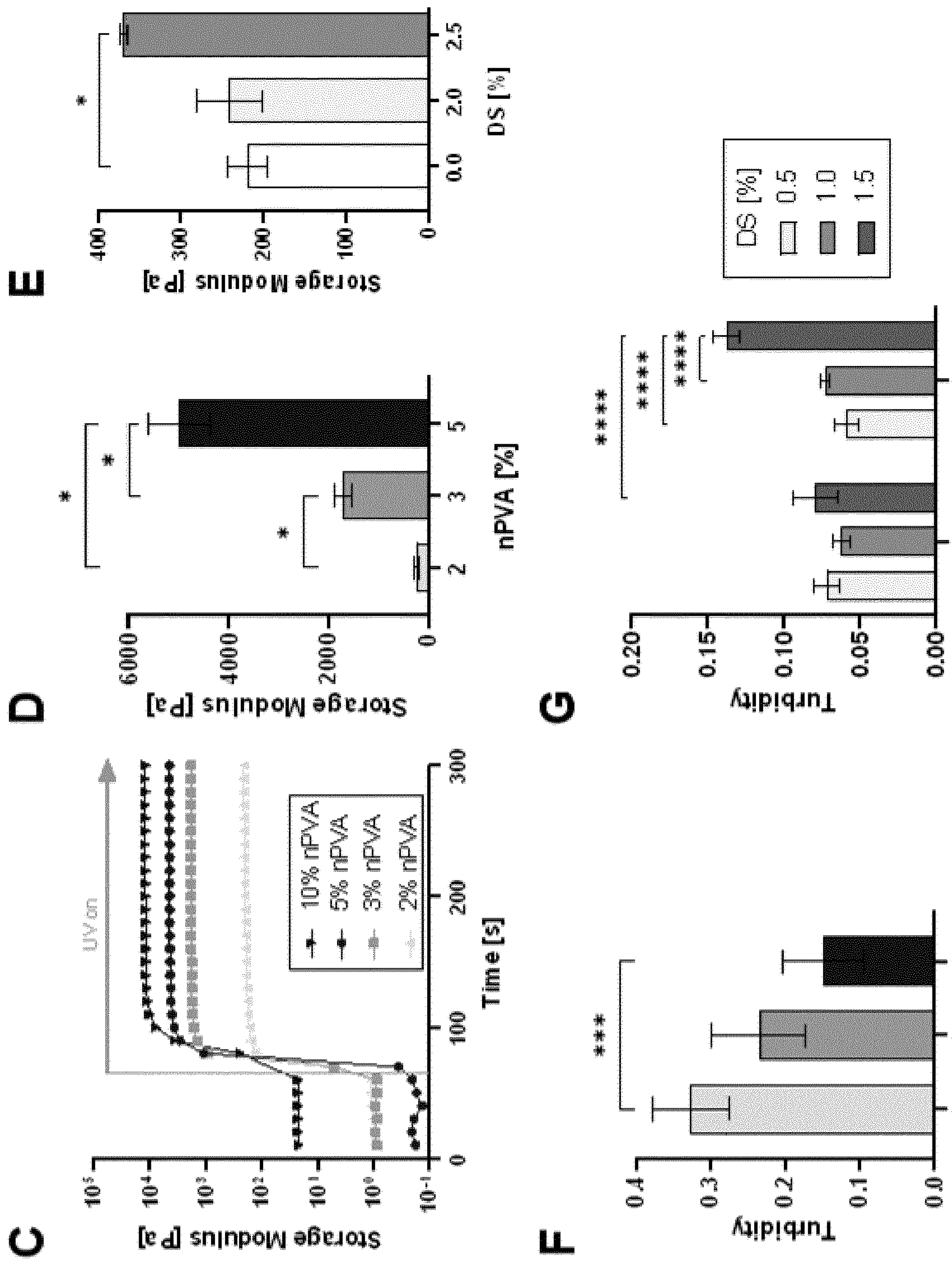

Fig. 4

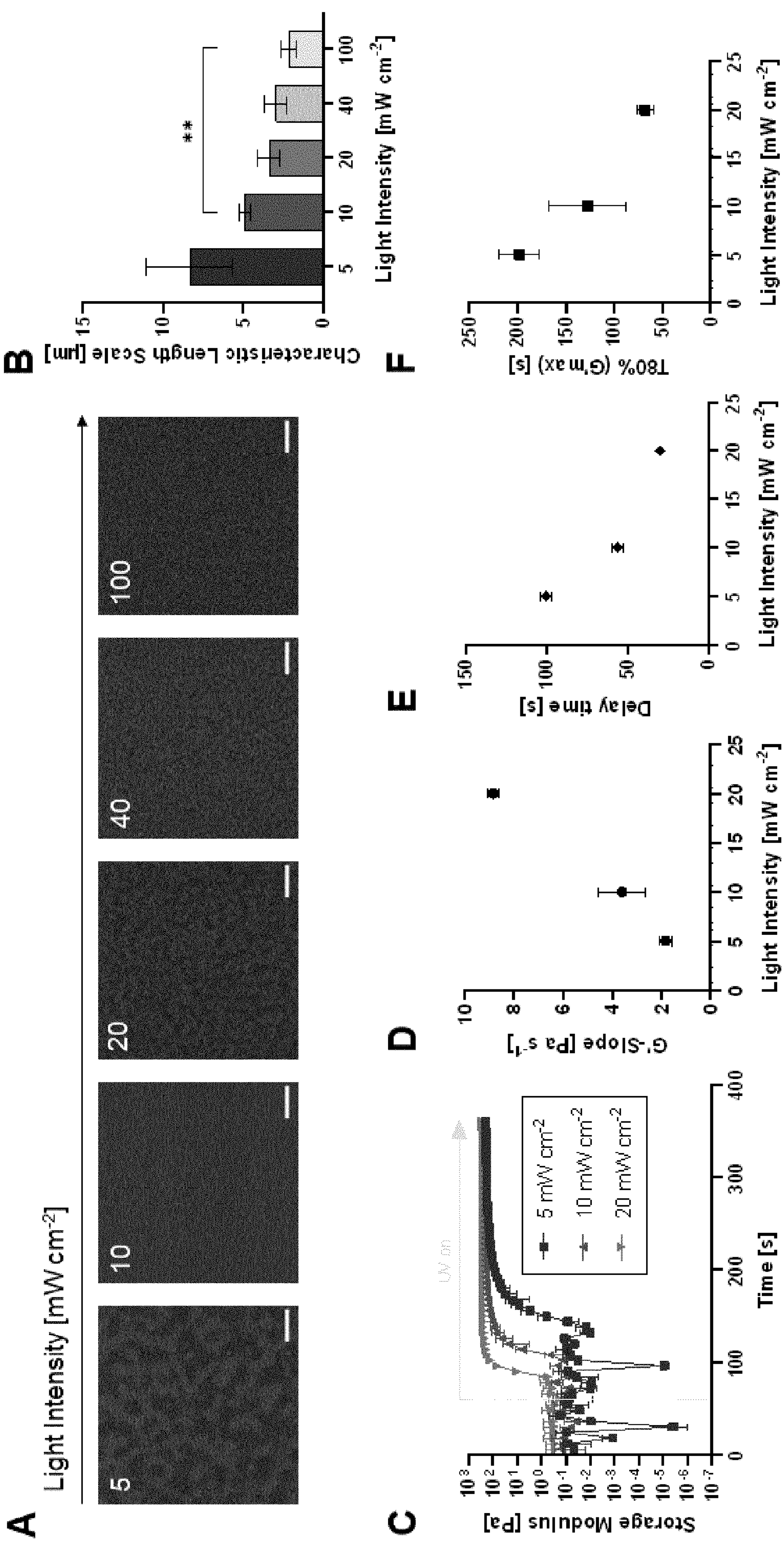
A
Light Intensity [mW cm-2]
5
10
20
40
100
B
Characteristic Length Scale [µm]
**
Light Intensity [mW cm-2]
C
Storage Modulus [Pa]
Time [s]
UV on
5 mW cm-2
10 mW cm-2
20 mW cm-2
D
G'-Slope [Pa s-1]
Light Intensity [mW cm-2]
E
Delay time [s]
Light Intensity [mW cm-2]
F
T80% (G'max) [s]
Light Intensity [mW cm-2]

Fig. 5

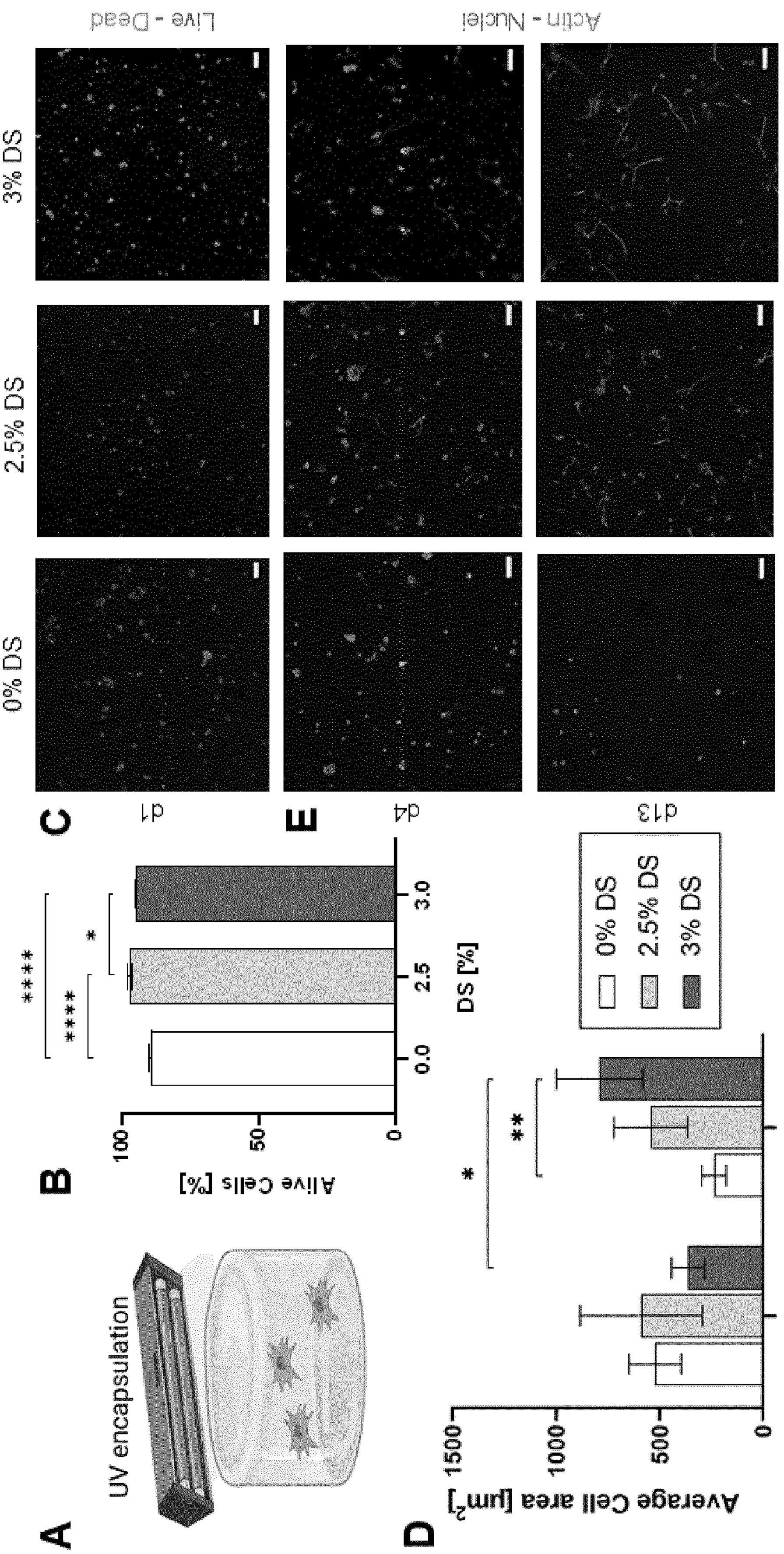
A
UV encapsulation
B
Alive Cells [%]
100
50
0
0.0
2.5
3.0
DS [%]
****
****
*
C
0% DS
2.5% DS
3% DS
d1
Live - Dead
D
Average Cell area [µm²]
1500
1000
500
0
d4
d13
*
**
0% DS
2.5% DS
3% DS
E
d4
d13
Actin - Nuclei

Fig. 6

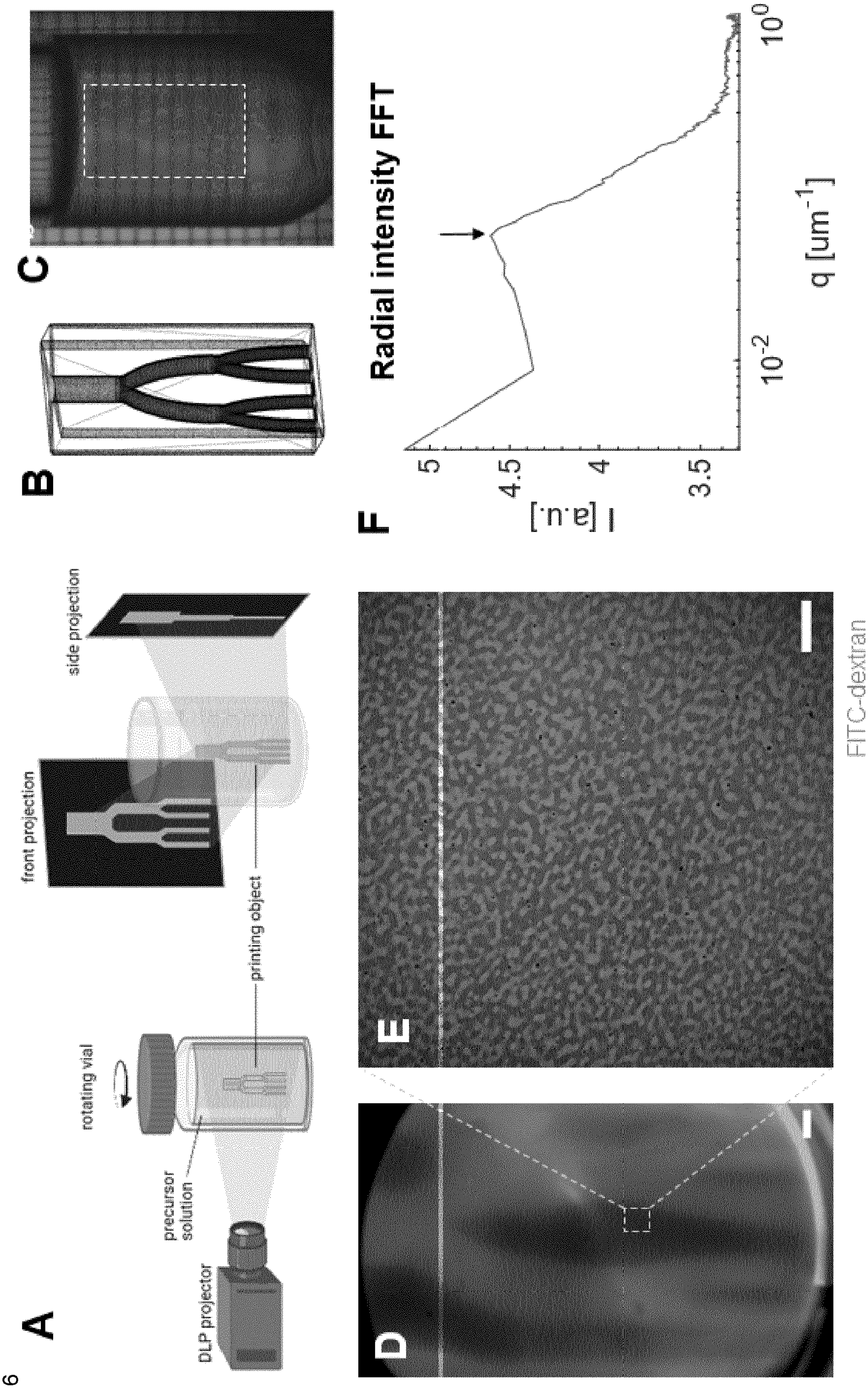
A
DLP projector
precursor solution
rotating vial
printing object
front projection
side projection
B
C
D
E
FITC-dextran
F
Radial intensity FFT
I [a.u.]
5
4.5
4
3.5
q [um-1]
10-2
100

Fig. 7

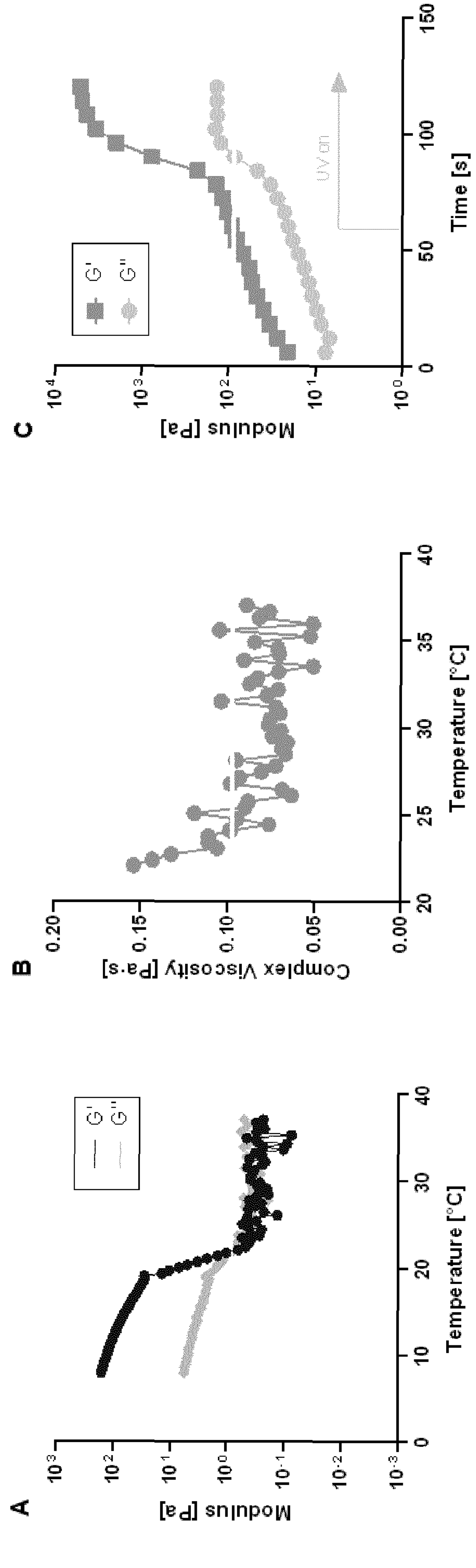
A
Modulus [Pa]
Temperature [°C]
G'
G''
B
Complex Viscosity [Pa·s]
Temperature [°C]
C
Modulus [Pa]
Time [s]
G'
G''
UV on

Fig. 8

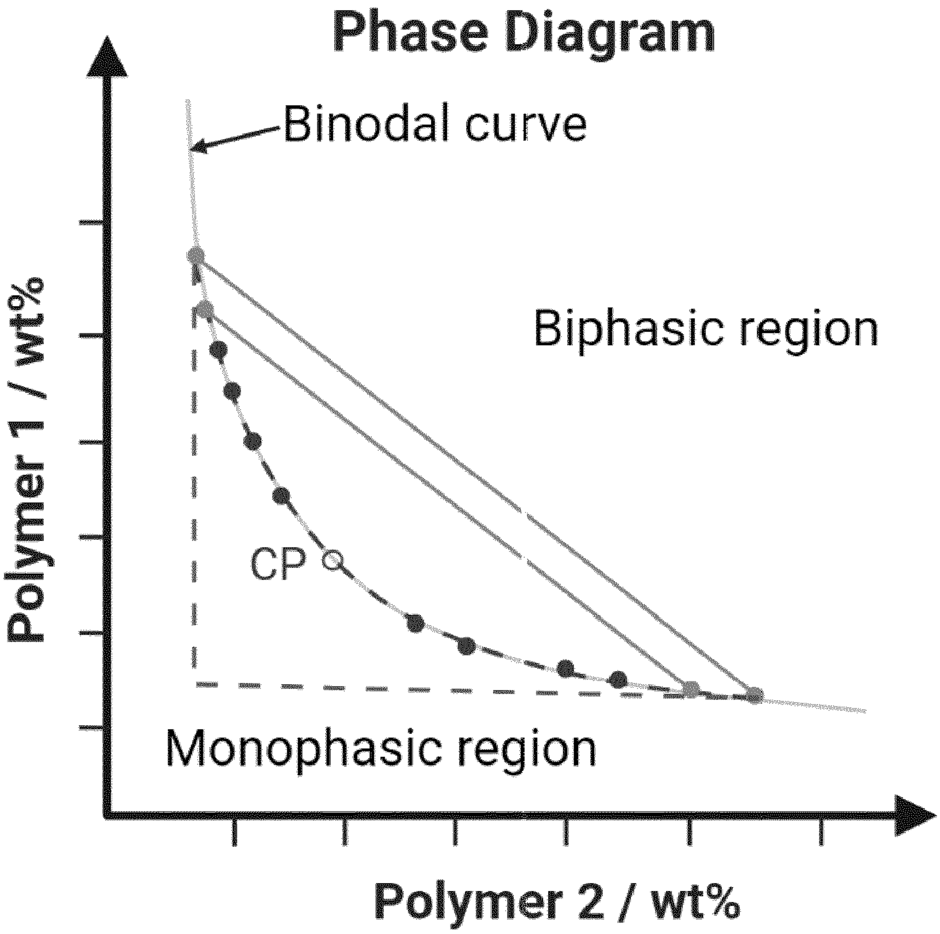
Phase Diagram
Binodal curve
Biphasic region
CP
Monophasic region
Polymer 1 / wt%
Polymer 2 / wt%

Fig. 9

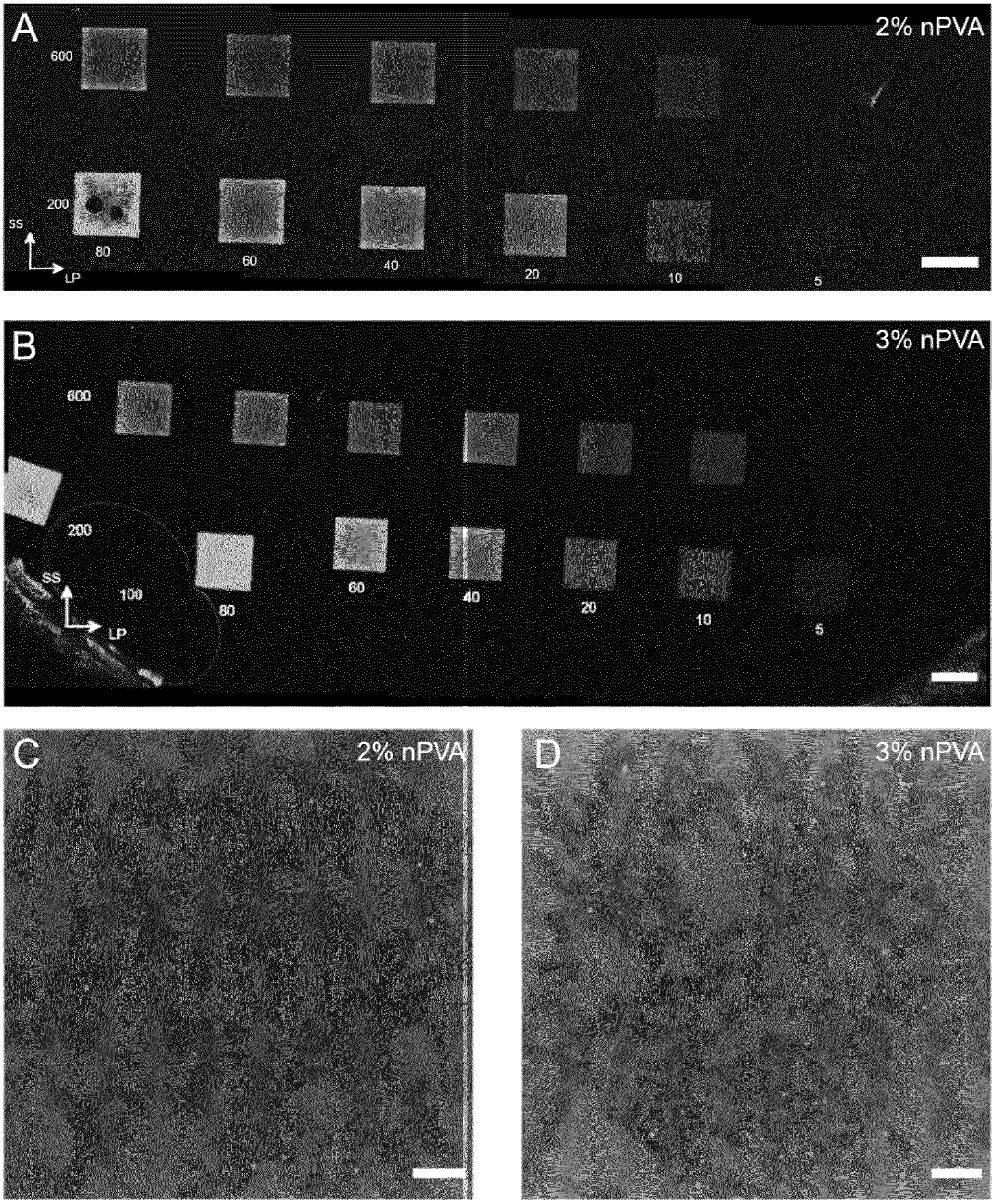
A
2% nPVA
600
200
SS
LP
80
60
40
20
10
5
B
3% nPVA
600
200
SS
100
LP
80
60
40
20
10
5
C
2% nPVA
D
3% nPVA

Fig. 10

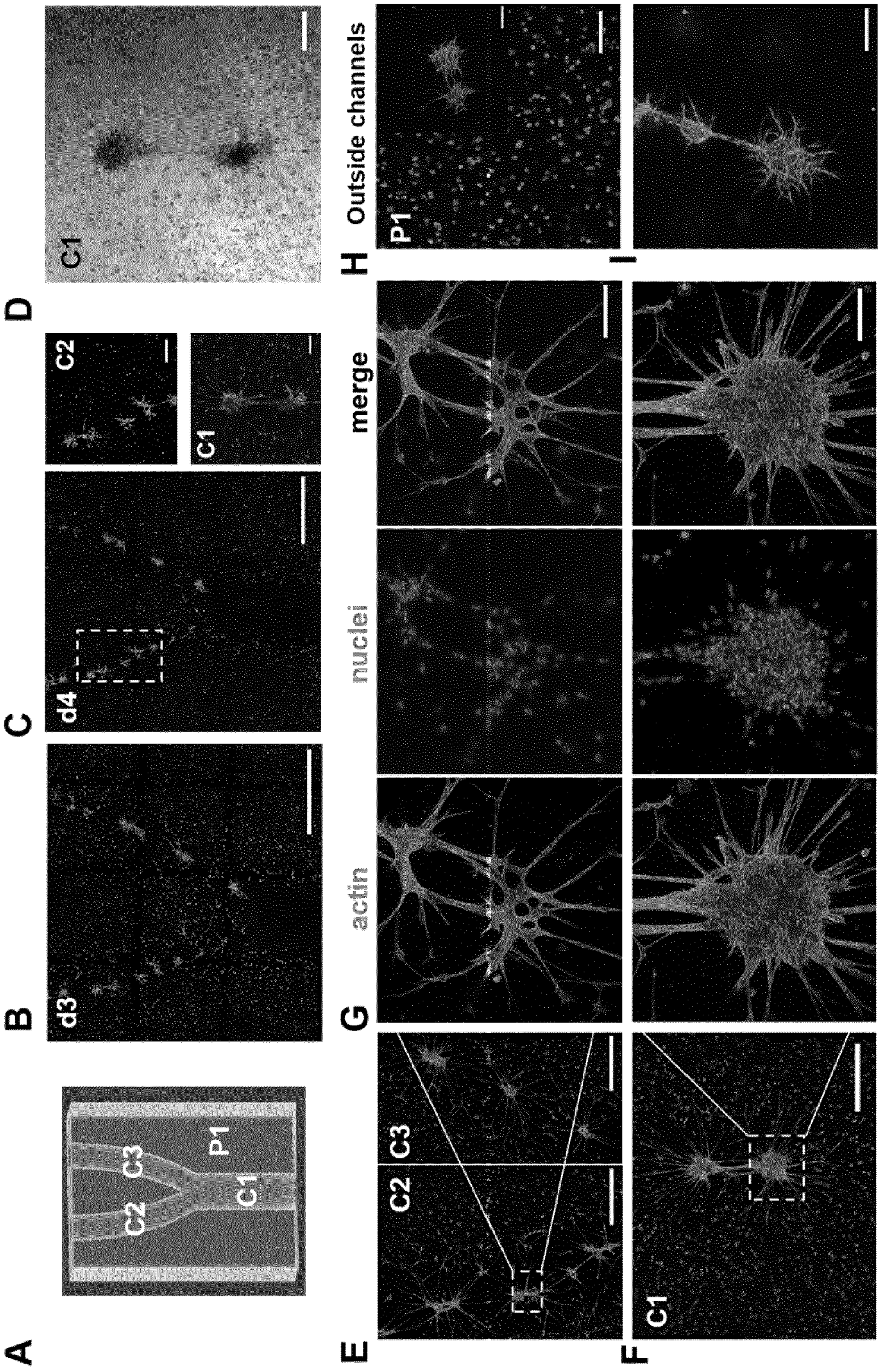
A
C2
C3
P1
C1
B
d3
C
d4
C2
C1
D
C1
E
C2
C3
F
C1
G
actin
nuclei
merge
H
P1
Outside channels
I

Fig. 11

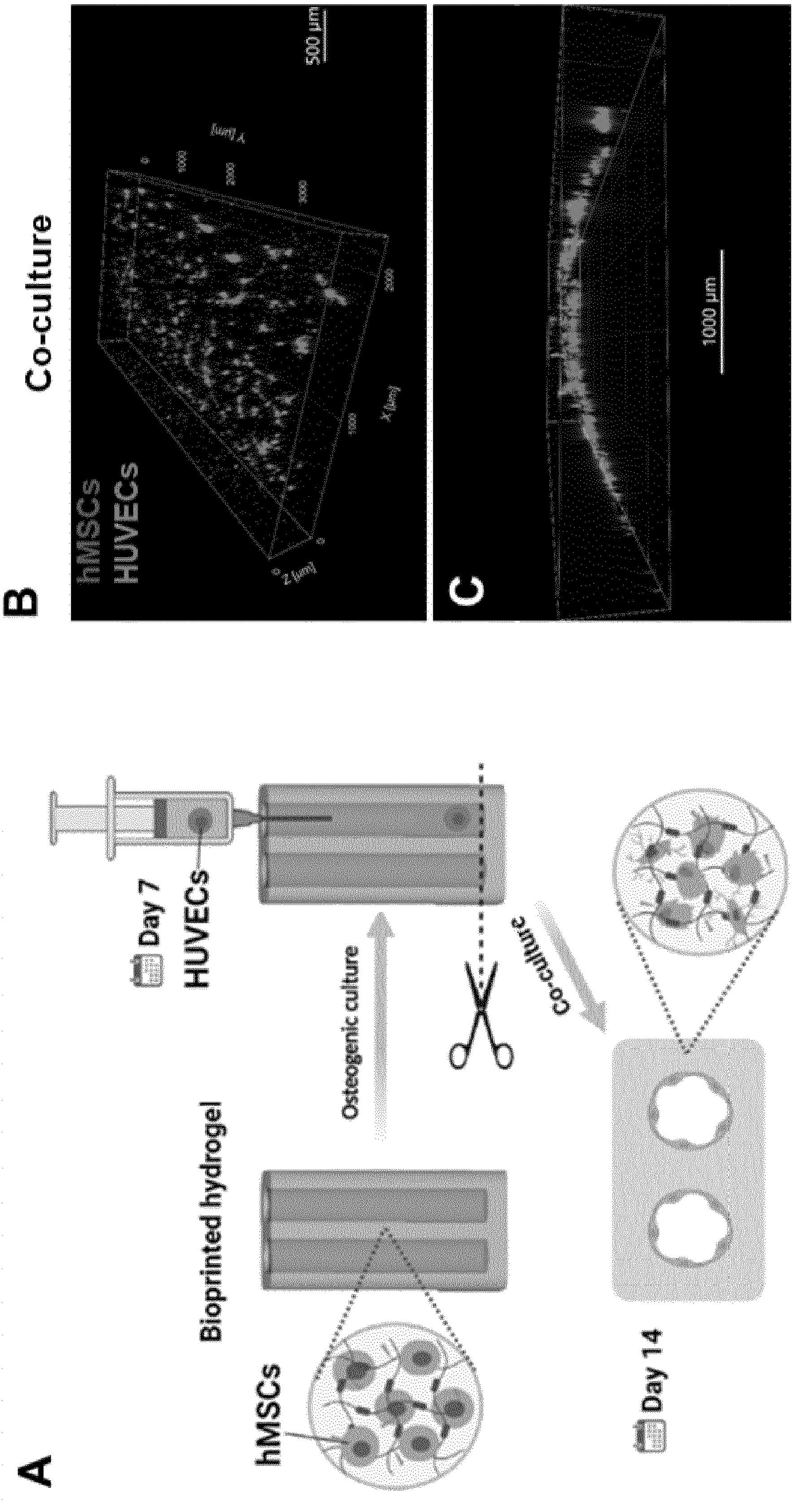
A
hMSCs
Bioprinted hydrogel
Osteogenic culture
Day 7
HUVECs
Co-culture
Day 14
B
Co-culture
hMSCs
HUVECs
500 µm
C
1000 µm

Fig. 12

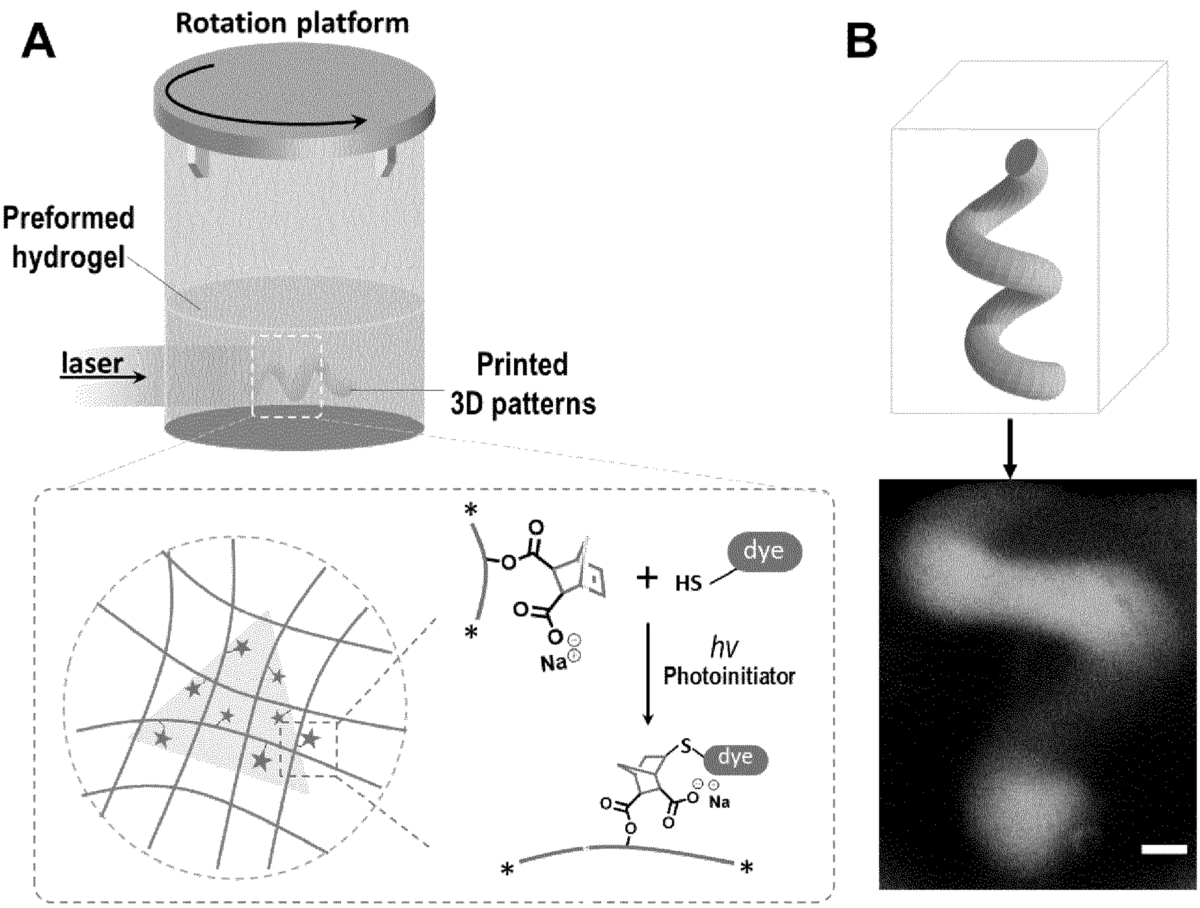
A
Rotation platform
Preformed hydrogel
laser
Printed 3D patterns
dye
HS
hv
Photoinitiator
S
dye
B

Fig. 13

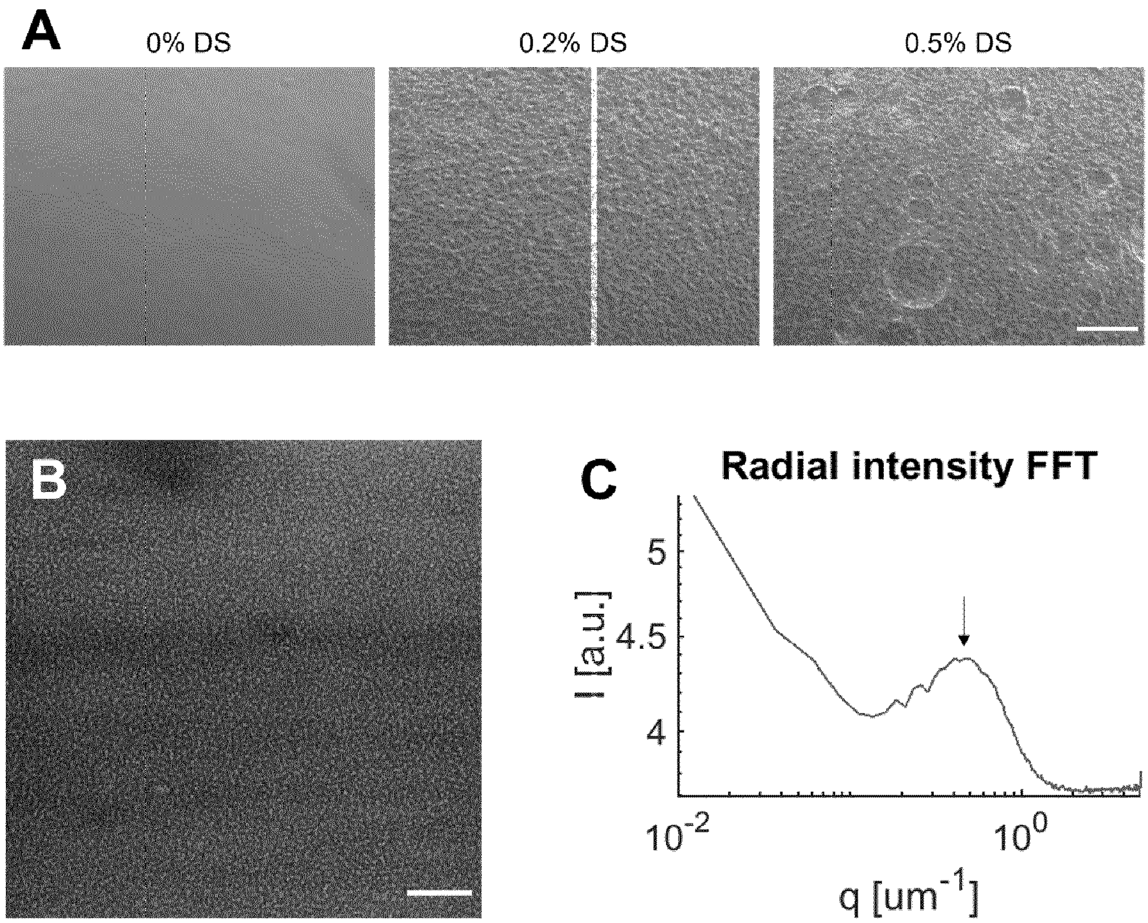

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017001451 A1
- WO 2015002707 A1
- EP 3344299 B1
- US 2018243465 A1
- US 20200384682 A1
- EP 3868416 A1


### Non-patent literature cited in the description


- **MOORE et al.** *Nat Mater*, 2020, vol. 19 (2), 212-217
- **DONG et al.** *Nature Communications*, 2021, vol. 12 (1), 247
- **MAYER et al.** *Advanced Materials*, 2020, vol. 32 (32), 2002044
- **KLEGER et al.** *Scientific Reports*, 2021, vol. 11 (1), 22316
- **BROGUIERE et al.** *Biomaterials*, 2019, vol. 200, 56-65
- **SAMBROOK et al.** Molecular Cloning: A Laboratory Manual. Cold Spring Harbor Laboratory Press, 2012
- **AUSUBEL et al.** Short Protocols in Molecular Biology. John Wiley & Sons, Inc., 2002
- *CHEMICAL ABSTRACTS*, 61551-69-7
- **BENEDIKT et al.** *J. Polymer Science Part A Polymer Chemistry*, 2015, vol. 54, 473-479, https://doi.ora/10.1002/pola.27903
- **TROMAYER et al.** *Polymer Chemistry*, 2018, vol. 9, 3108-3117, https://doi.org/10.1039/C8PY00278A
- **XIAO et al.** *Biomacromolecules*, 2002, vol. 3 (6), 1304-1311
- **URCIUOLO et al.** *Nat Biomed Eng*, 2020, vol. 4, 901-915
- **PARTLOW et al.** *ACS Biomater. Sci. Eng.*, 2016, vol. 2 (12), 2108-2121
- **LOEBEL et al.** *Biomacromolecules*, 2017, vol. 18 (3), 855-864
- **SCINTO et al.** Bioorthogonal chemistry. *Nat Rev Methods Primers*, 2021, vol. 1, 30, https://doi.orq/10.1038/s43586-021-00028-z
- **ADZIMA et al.** *Nature Chem*, 2011, vol. 3, 256-259, https://doi.orq/10.1038/nchem.980
- **PENG et al.** *Chem. Mater.*, 2014, vol. 26 (23), 6819-6826
- **ZHANG et al.** *ACS Macro Lett.*, 2016, vol. 5 (2), 229-233
- **QIN et al.** *Advanced Functional Materials*, 2015, vol. 25, 6606-6617, https://doi.org/10.1002/adfm.201501637
- **BAUDIS et al.** *J Polymer Science*, 2016, vol. 54, 2060-2070, https://doi.org/10.1002/pola.28073
- **BENMOUNA et al.** *Macromolecules*, 2000, vol. 33 (3), 960-967
- **CAPELA et al.** *Chem Eng Educ.*, 2019, vol. 53 (2), 112-120
- **HUEBSCH et al.** *Nature Mater*, 2015, vol. 14, 1269-1277, https://doi.org/10.1038/nmat4407
- **TRAN-CONG et al.** *Physical Review*, 1999, vol. E 60 (2), R1150-1153
- **VANDAELE et al.** *Soft Matter*, 2020, vol. 16, 4210-4219
- **GEHLEN et al.** *bioRxiv*
- **KIMURA et al.** *Soft Matter*, 2013, vol. 9 (35), 8428-8437
- **BUTLER** ; **HEPPENSTALL-BUTLER**. *Biomacromolecules*, 2003, vol. 4, 928-936, https://doi.org/10.1021/bm0340319
- **WANG et al.** *Adv Funct Mater*, 2020, vol. 28 (45), 1804107
- **QIN et al.** *Adv Mater*, 2018, vol. 30, 1705564
- **VANDAELE et al.** *Soft Matter*, 2020, vol. 16 (17), 4210-4219